# EUROPEAN PATENT SPECIFICATION

(11) **EP 1 272 651 B1**
(45) Date of publication and mention of the grant of the patent: **06.02.2008**
(21) Application number: 01930493.0
(22) Date of filing: 13.04.2001
(51) Int. Cl.: C12N 15/82, C12N 15/29, C07K 14/415, A01H 5/00

(54) **CONTROL OF FRUIT DEHISCENCE IN ARABIDOPSIS BY INDEHISCENT1 GENES**
KONTROLLE DER FRUCHT-DEHISZENZ IN ARABIDOPSIS DURCH INDEHISCENT1-GENE
MAITRISE DE LA DEHISCENCE DU FRUIT DANS DES ARABIDOPSIS AU MOYEN DE GENES 1 INDEHISCENTS

(30) Priority: 13.04.2000 US 548971
(43) Date of publication of application: 08.01.2003
(60) Divisional application: 07023350.7
(73) Proprietor: The Regents of the University of California, La Jolla, CA 92093-0910 (US)
(72) Inventor: LILJEGREN, Sarah, La Jolla, CA 92037 (US); YANOFSKY, Martin, F., San Diego, CA 92130 (US)
(74) Representative: Brasnett, Adrian Hugh
(86) International application number: PCT/US2001/011967
(87) International publication number: WO 2001/079517

(56) References cited:
- WO-A-96/30529
- WO-A-97/13865
- WO-A-99/00502
- WO-A-99/49046
- DATABASE EMBL SEQUENCE LIBRARY [Online] 16 March 2000 (2000-03-16) EU ARABIDOPSIS SEQUENCING PROJECT: "Arabidopsis thaliana DNA chromosome 4, contig fragment No. 1" XP002187880
- DATABASE TREMBL DATABASE [Online] 1 November 1998 (1998-11-01) RYAN,E., EDWARDS,J., PAPE,K., : "The sequence of A. thaliana F6N15" XP002187881
- DATABASE EMBL SEQUENCE LIBRARY [Online] 13 October 1997 (1997-10-13) ROUNSLEY S.D., KELLEY J.M., FIELD C.E., CRAVEN M.B., ADAMS M.D., VENTER J.C.: "Use of a BAC End Sequence Database To Identify Minimal Overlaps for Arabidopsis Genomic Sequencing - F2G15TF IGF Arabidopsis thaliana genomic clone F2G15, genomic survey sequence" XP002187882
- DATABASE EMBL SEQUENCE LIBRARY [Online] 31 January 2000 (2000-01-31) BUELL C.R., ET AL.: "Genomic survey sequencing of Landsberg erecta ecotype of Arabidopsis thaliana and identification of sequence-based polymorphisms - LERAM13TR LERA Arabidopsis thaliana genomic clone LERAM13, genomic survey sequence" XP002187883
- SUNDARESAN V ET AL: "PATTERNS OF GENE ACTION IN PLANT DEVELOPMENT REVEALED BY ENHANCER TRAP AND GENE TRAP TRANSPOSABLE ELEMENTS" GENES AND DEVELOPMENT, COLD SPRING HARBOR, NY, US, vol. 9, no. 14, 15 July 1995 (1995-07-15), pages 1797-1810, XP000674520 ISSN: 0890-9369 cited in the application

## Description

### STATEMENT AS TO RIGHTS TO INVENTIONS MADE UNDER FEDERALLY SPONSORED RESEARCH AND DEVELOPMENT

This invention was made with Government support under National Science Foundation Grant number IBN-9985530. The Government has certain rights in the invention.

### FIELD OF THE INVENTION

This invention relates plant genetic engineering. In particular, the invention relates to methods and compositions that modulate fruit dehiscence in plants.

### BACKGROUND OF THE INVENTION

Rapeseed is one of the most important oilseed crops after soybeans and cottonseed, representing 10% of the world oilseed production in 1990. Rapeseed contains 40% oil, which is pressed from the seed, leaving a high-protein seed meal of value for animal feed and nitrogen fertilizer. Rapeseed oil, also known as canola oil, is a valuable product, representing the fourth most commonly traded vegetable oil in the world.

Unfortunately, the yield of seed from rapeseed and related plants is limited by pod dehiscence, which is a process that occurs late in fruit development whereby the pod is opened and the enclosed seeds released. Degradation and separation of cell walls along a discrete layer of cells dividing the two halves of the pod, termed the "dehiscence zone," result in separation of the two halves of the pod and release of the contained seeds. The dehiscence zone is a region of only one to three cells in width that extends along the entire length of the valve/replum boundary (Meakin and Roberts, J. Exp. Botany 41:995-1002 (1990)). As the cells in the dehiscence zone separate from one another, the valves detach from the replum, allowing seeds to be dispersed. Seed "shattering," whereby seeds are prematurely shed through dehiscence before the crop can be harvested, is a significant problem faced by commercial seed producers and represents a loss of income to the industry. Adverse weather conditions can exacerbate the process of dehiscence, resulting in greater than 50% loss of seed yield.

The fruit, a complex structure unique to flowering plants, mediates the maturation and dispersal of seeds. In most flowering plants, the fruit consists of the pericarp, which is derived from the ovary wall, and the seeds, which develop from fertilized ovules. *Arabidopsis,* which is typical of the more than 3000 species of the *Brassicaceae,* produces fruit in which the two carpel valves (ovary walls) are joined to the replum, a visible suture that divides the two carpels.

The plant hormone ethylene is produced by developing seeds and appears to be an important regulator of the dehiscence process. One line of evidence supporting a role for ethylene in regulation of dehiscence comes from studies of fruit ripening, which, like fruit dehiscence, is a process involving the breakdown of cell wall material. In fruit ripening, ethylene acts in part by activating cell wall degrading enzymes such as polygalacturonase (Theologis et al., Develop. Genetics 14:282-295 (1993)). Moreover, in genetically modified tomato plants in which the ethylene response is blocked, such as transgenic tomato plants expressing antisense polygalacturonase, there is a significant delay in fruit ripening (Lanahan et al., The Plant Cell 6:521-530 (1994); Smith et al., Nature 334:724-726 (1988)).

In dehiscence, ultrastructural changes that culminate in degradation of the middle lamella of dehiscence zone cell walls weaken rapeseed pods and eventually lead to pod shatter. As in fruit ripening, hydrolytic enzymes including polygalacturonases play a role in this programmed breakdown. For example, in oilseed rape, a specific endo-polygalacturonase, RDPG1, is upregulated and expressed exclusively in the dehiscence zone late in pod development (Petersen et al., Plant Mol. Biol. 31:517-527 (1996), which is incorporated herein by reference). Ethylene may regulate the activity of hydrolytic enzymes involved in the process of dehiscence as it does in fruit ripening (Meakin and Roberts, J. Exp. Botany 41:1003-1011 (1990), which is incorporated herein by reference). Yet, until now, the proteins that control the process of dehiscence, such as those regulating the relevant hydrolytic enzymes, have eluded identification.

Attempts to solve the problem of pod shatter and early fruit dehiscence over the past 20 years have focused on the breeding of shatter-resistant varieties. However, these plant hybrids are frequently sterile and lose favorable characteristics that must be regained by backcrossing, which is both time-consuming and laborious. Other strategies to alleviate pod shattering include the use of chemicals such as pod sealants or mechanical techniques such as swathing to reduce wind-stimulated shattering. To date, however, a simple method for producing genetically modified plants that do not open and release their seeds prematurely has not been described.

Thus, a need exists for identifying genes that regulate the dehiscence process and for developing genetically modified plant varieties in which the natural seed dispersal process is delayed. The present invention satisfies this need and provides related advantages as well.

### SUMMARY OF THE INVENTION

The present invention provides an expression cassette comprising a promoter operably linked to an IND1 polynucleotide sequence, or complement thereof, encoding an IND1 polypeptide at least about 70% identical to SEQ ID NO:1. The expression cassette, for instance, can comprise a polynucleotide that encodes SEQ ID NO:2. In another embodiment, the expression cassette, for instance, can comprise positions from about 2765 to about 3361 of SEQ ID NO:1. For example, the expression cassette can comprise SEQ ID NO:1. In some embodiments, the expression cassette comprises a promoter. The promoter, for instance, can be constitutive or tissue specific. In one aspect of the invention, the promoter is a dehiscence zone specific promoter. In another aspect, the promoter can comprise positions from about 1 to about 2764 or positions from about 3362 to about 3856 of SEQ ID NO:1.

The present invention also provides a plant comprising a recombinant expression cassette comprising a promoter operably linked to a polynucleotide sequence encoding an IND1 polypeptide at least about 70% identical to SEQ ID NO:1. In one aspect, the polynucleotide sequence encoding the IND1 polypeptide is operably linked to the promoter in the antisense orientation. In another aspect, the polynucleotide sequence encoding the IND1 polypeptide is operably linked to the promoter in the sense orientation. The polynucleotide sequence can further comprise a second polynucleotide sequence encoding the IND1 polypeptide wherein the second polynucleotide sequence is operably linked to a second promoter in the antisense orientation. In some embodiments, the plant of the invention has reduced lignification in valve margin cells. In other embodiments, the promoter is a dehiscence zone-selective regulatory element. In some of these embodiments, the regulatory element comprises positions from about 1 to about 2764 or from about 3362 to about 3856 of SEQ ID NO:1.

The present invention also provides a method of delaying fruit dehiscence in a plant comprising suppressing expression of an IND1 nucleic acid in the plant by introducing into the plant a recombinant expression cassette comprising a promoter operably linked to a polynucleotide sequence encoding an IND1 polypeptide at least 70% identical to SEQ ID NO:2. In some embodiments, the IND 1 polypeptide is SEQ ID NO:2. In other embodiments, the IND1 polynucleotide comprises positions from about 2765 to about 3361 of SEQ ID NO:1. In one aspect of the invention, the IND1 polynucleotide comprises SEQ ID NO:1. The method can include a polynucleotide sequence encoding the IND1 polypeptide operably linked to the promoter in the antisense orientation. In another embodiment, the promoter is linked to the promoter is the sense orientation. The polynucleotide sequence can further comprise a second polynucleotide sequence encoding the IND1 polypeptide wherein the second polynucleotide sequence is operably linked to a second promoter in the antisense orientation. In some embodiments, the method results in a plant with reduced lignification in valve margin cells. In other embodiments, the promoter is a dehiscence zone-selective regulatory element. In some of these embodiments, the regulatory element comprises positions from about 1 to about 2764 or from about 3362 to about 3856 of SEQ ID NO:1. In one aspect, the recombinant expression cassette is introduced into the plant with *Agrobacterium.*

The present invention also provides a method of delaying fruit dehiscence in a plant comprising suppressing expression of an IND1 gene in the plant by introducing into the plant a recombinant expression cassette comprising a polynucleotide sequence at least about 70% identical to positions from about 1 to about 2764 or from about 3362 to about 3856 of SEQ ID NO: 1. In one aspect of the invention, the polynucleotide sequence comprises positions from about 1 to about 2764 or from about 3362 to about 3856 of SEQ ID NO:1. In some aspects, lignification is reduced in valve margin cells.

### DEFINITIONS

The phrase "nucleic acid" refers to a single or double-stranded polymer of deoxyribonucleotide or ribonucleotide bases read from the 5' to the 3' end. Nucleic acids may also include modified nucleotides that permit correct read through by a polymerase and do not alter expression of a polypeptide encoded by that nucleic acid.

The phrase "polynucleotide sequence" or "nucleic acid sequence" includes both the sense and antisense strands of a nucleic acid as either individual single strands or in the duplex. It includes, but is not limited to, self-replicating plasmids, chromosomal sequences, and infectious polymers of DNA or RNA.

The phrase "nucleic acid sequence encoding" refers to a nucleic acid which, directs the expression of a specific protein or peptide. The nucleic acid sequences include both the DNA strand sequence that is transcribed into RNA and the RNA sequence that is translated into protein. The nucleic acid sequences include both the full length nucleic acid sequences as well as non-full length sequences derived from the full length sequences. It should be further understood that the sequence includes the degenerate codons of the native sequence or sequences which may be introduced to provide codon preference in a specific host cell.

The term "promoter" or "regulatory element" refers to a region or sequence determinants located upstream or downstream from the start of transcription and which are involved in recognition and binding of RNA polymerase and other proteins to initiate transcription. A "plant promoter" is a promoter capable of initiating transcription in plant cells. Such promoters need not be of plant origin, for example, promoters derived from plant viruses, such as the CaMV35S promoter, can be used in the present invention.

As used herein, the term "dehiscence zone-selective regulatory element" refers to a nucleotide sequence that, when operatively linked to a nucleic acid molecule, confers selective expression upon the operatively linked nucleic acid molecule in a limited number of plant tissues, including the valve margin or dehiscence zone. The valve margin is the future site of the dehiscence zone and encompasses the margins of the outer replum as well as valve cells adjacent to the outer replum. The dehiscence zone, which develops in the region of the valve margin, refers to the group of cells that separate during the process of dehiscence, allowing valves to come apart from the replum and the enclosed seeds to be released. Thus, a dehiscence zone-selective regulatory element, as defined herein, confers selective expression in the mature dehiscence zone, or confers selective expression in the valve margin, which marks the future site of the dehiscence zone.

A dehiscence zone-selective regulatory element can confer specific expression exclusively in cells of the valve margin or dehiscence zone or can confer selective expression in a limited number of plant cell types including cells of the valve margin or dehiscence zone. A *SHATTERPROOF1 or SHATTERPROOF2 (SHP1* and *SHP2,* also designated as *AGL1* and *AGL5,* repectively) regulatory element, for example, which confers selective expression in ovules and placenta as well as in the dehiscence zone, is a dehiscence zone-selective regulatory element as defined herein. Similarly, an IND1 regulatory element also confers selective expression in the dehiscence zone. A dehiscence zone-selective regulatory element generally is distinguished from other regulatory elements by conferring selective expression in the valve margin or dehiscence zone without conferring expression throughout the adjacent carpel valves.

It is understood that limited modifications can be made without destroying the biological function of a regulatory element and that such limited modifications can result in dehiscence zone-selective regulatory elements that have substantially equivalent or enhanced function as compared to a wild type IND1 regulatory element. These modifications can be deliberate, as through site-directed mutagenesis, or can be accidental such as through mutation in hosts harboring the regulatory element. All such modified nucleotide sequences are included in the definition of a dehiscence zone-selective regulatory element as long as the ability to confer selective expression in the valve margin or dehiscence zone is substantially retained.

The term "plant" includes whole plants, shoot vegetative organs/structures (*e.g.* leaves, stems and tubers), roots, flowers and floral organs/structures (*e.g*. bracts, sepals, petals, stamens, carpels, anthers and ovules), seed (including embryo, endosperm, and seed coat) and fruit (the mature ovary), plant tissue (*e.g*. vascular tissue, ground tissue, and the like) and cells (*e.g*. guard cells, egg cells, trichomes and the like), and progeny of same. The class of plants that can be used in the method of the invention is generally as broad as the class of higher and lower plants amenable to transformation techniques, including angiosperms (monocotyledonous and dicotyledonous plants), gymnosperms, ferns, and multicellular algae. It includes plants of a variety of ploidy levels, including aneuploid, polyploid, diploid, haploid and hemizygous.

The term "seed plant" means an angiosperm or gymnosperm. An angiosperm is a seed-bearing plant whose seeds are borne in a mature ovary (fruit). An angiosperm commonly is recognized as a flowering plant. Angiosperms are divided into two broad classes based on the number of cotyledons, which are seed leaves that generally store or absorb food. Thus, a monocotyledonous angiosperm is an angiosperm having a single cotyledon, whereas a dicotyledonous angiosperm is an angiosperm having two cotyledons. A variety of angiosperms are known including, for example, oilseed plants, leguminous plants, fruit-bearing plants, ornamental flowers, cereal plants and hardwood trees, which general classes are not necessarily exclusive. The skilled artisan will recognize that the methods of the invention can be practiced using these or other angiosperms, as desired. A gymnosperm is a seed-bearing plant with seeds not enclosed in an ovary.

The phrase "host cell" refers to a cell from any organism. Preferred host cells are derived from plants, bacteria, yeast, fungi, insects or other animals. Methods for introducing polynucleotide sequences into various types of host cells are well known in the art.

The term "delayed," as used herein in reference to the timing of seed dispersal in a fruit produced by a non-naturally occurring plant of the invention, means a significantly later time of seed dispersal as compared to the time seeds normally are dispersed from a corresponding plant at the same developmental stage expressing naturally-occurring levels of IND1. Thus, the term "delayed" is used broadly to encompass both seed dispersal that is significantly postponed as compared to the seed dispersal in a corresponding plant, and to seed dispersal that is completely precluded, such that fruits never release their seeds unless there is human or other intervention.

It is recognized that there can be natural variation of the time of seed dispersal within a plant species or variety. However, a "delay" in the time of seed dispersal in a non-naturally occurring plant of the invention readily can be identified by sampling a population of the non-naturally occurring plants and determining that the normal distribution of seed dispersal times is significantly later, on average, than the normal distribution of seed dispersal times in a population of the corresponding plant species or variety that does not contain an exogenous IND1 polynucleotide. Thus, production of non-naturally occurring plants of the invention provides a means to skew the normal distribution of the time of seed dispersal from pollination, such that seeds are dispersed, on average, at least about 1%, 2%, 5%, 10%, 30%, 50%, 100%, 200% or 500% later than in the corresponding plant species that does not contain an exogenous nucleic acid molecule encoding an IND 1 gene product.

The term "suppressed" or "decreased" encompasses the absence of IND1 protein in a plant, as well as protein expression that is present but reduced as compared to the level of IND1 protein expression in a wild type plant. Furthermore, the term suppressed refers to IND 1 protein expression that is reduced throughout the entire domain of IND1 expression, or to expression that is reduced in some part of the IND1 expression domain, provided that the resulting plant is characterized by delayed seed dispersal. The term "suppressed" also encompasses an amount of IND1 protein that is equivalent to wild type IND1 expression, but where the IND1 protein has a reduced level of activity. As discussed above, IND1 each contain a conserved an basic HLH domain; point mutations or gross deletions within the HLH domain that reduce the DNA-binding activity of IND1 can reduce or destroy the activity of IND and, therefore, "suppress" IND 1 expression as defined herein. One skilled in the art will recognize that, preferably, IND1 expression is essentially absent in the valve margin of a plant or the IND1 protein is essentially non-functional.

"Increased" or "enhanced" IND 1 activity or expression of a *IND1* gene refers to an augmented change in IND1 activity. Examples of such increased activity or expression include the following. IND1 activity or expression of the *IND1* gene is increased above the level of that in wild-type, non-transgenic control plants (i.e. the quantity of IND1 activity or expression of the *IND1* gene is increased). IND1 activity or expression of the *IND1* gene is in an organ, tissue or cell where it is not normally detected in wild-type, non-transgenic control plants (i.e. spatial distribution of IND1 activity or expression of the *IND1* gene is increased). IND1 activity or expression is increased when IND1 activity or expression of the *IND1* gene is present in an organ, tissue or cell for a longer period than in a wild-type, non-transgenic controls (i.e. duration of IND1 activity or expression of the *IND1* gene is increased).

A polynucleotide sequence is "heterologous to" a second polynucleotide sequence if it originates from a foreign species, or, if from the same species, is modified by human action from its original form. For example, a promoter operably linked to a heterologous coding sequence refers to a coding sequence from a species different from that from which the promoter was derived, or, if from the same species, a coding sequence which is different from any naturally occurring allelic variants.

A polynucleotide "exogenous to" an individual plant is a polynucleotide which is introduced into the plant, or a predecessor generation of the plant, by any means other than by a sexual cross. An exogenous nucleic acid molecule can have a naturally occurring or non-naturally occurring nucleotide sequence and can be a heterologous nucleic acid molecule derived from a different plant species than the plant into which the nucleic acid molecule is introduced or can be a nucleic acid molecule derived from the same plant species as the plant into which it is introduced. Examples of means by which this can be accomplished are described below, and include Agrobacterium-mediated transformation, biolistic methods, electroporation, in planta techniques, and the like.

An *"IND1* polynucleotide" is a nucleic acid sequence comprising (or consisting of) a coding region of about 50 to about 4000 nucleotides, sometimes from about 100 to about 3000 nucleotides and sometimes from about 200 to about 600 nucleotides, which hybridizes to SEQ ID NO:1 under stringent conditions (as defined below), or which encodes an IND1 polypeptide or fragment of at least 15 amino acids thereof. *IND1* polynucleotides can also be identified by their ability to hybridize under low stringency conditions (e.g., Tm ~40°C) to nucleic acid probes having the sequence of SEQ ID NO:1. SEQ ID NO: 1 is an example of a *IND1* polynucleotide.

A "promoter from a *IND1* gene" or *"IND1* promoter" will typically be about 500 to about 3000 nucleotides in length, usually from about 750 to 2750. Exemplary promoter sequences are shown as SEQ ID NO:3 and SEQ ID NO:4. SEQ ID NO:3 represents the 5' untranslated region of the *IND1* and SEQ ID NO:3 represents the 3' untranslated region of *IND1.* A IND1 promoter can also be identified by its ability to direct expression in the valve margin of fruit. In particular, the Ind1 promoeter directs expression at the valve margin of developing gynoecium just prior to fertilization (stage 13) through the maturation of the fruit (stage 17). The promoter does not provide significant expression in leaf tissue.

An "IND1 polypeptide" is a sequence of about 50 to about 200, sometimes 100 to 190, and preferably 198 amino acid residues encoded by a IND1 polynucleotide. IND1 polypeptides are characterized by the presence of an basic helix-loop-helix (HLH) domain which bind specific polynucleotide sequences. For instance amino acid residues ISDDPQTVVARRRRERISEKIRILKRIVPGGAKMDTASMLDEAIRYTKFLK represent the HLH domain of the polypeptide shown in SEQ ID NO:2. The HLH domain is known in the art and is shared by other transcription factors including uncharacterized sequences represented by GenBank accession number E1283552 and 2262147 and the gene product, PIF3 (Ni et al. Cell 95:657 (1998)). The HLH domain of IND1 is therefore a DNA binding domain.

As used herein, a homolog of a particular IND1 gene (e.g., SEQ ID NO:1) is a second gene in the same plant type or in a different plant type, which has a polynucleotide sequence of at least 50 contiguous nucleotides which are substantially identical (determined as described below) to a sequence in the first gene. It is believed that, in general, homologs share a common evolutionary past.

A "polynucleotide sequence from" a particular gene is a subsequence or full length polynucleotide sequence of an IND1 gene which, when present in a transgenic plant, has the desired effect. For example, one effect is inhibition of expression of the endogenous gene driving expression of an heterologous polynucleotide. A full length sequence of a particular gene disclosed here may contain about 95%, usually at least about 98% of an entire sequence shown in the Sequence Listing, below.

The term "reproductive tissues" as used herein includes fruit, ovules, seeds, pollen, pistols, flowers, or any embryonic tissue.

An "expression cassette" refers to a nucleic acid construct, which when introduced into a host cell, results in transcription and/or translation of a RNA or polypeptide, respectively. Antisense or sense constructs that are not or cannot be translated are expressly included by this definition.

In the case of both expression of transgenes and inhibition of endogenous genes (e.g., by antisense, or sense suppression) one of skill will recognize that the inserted polynucleotide sequence need not be identical and may be "substantially identical" to a sequence of the gene from which it was derived. As explained below, these variants are specifically covered by this term.

In the case where the inserted polynucleotide sequence is transcribed and translated to produce a functional polypeptide, one of skill will recognize that because of codon degeneracy a number of polynucleotide sequences will encode the same polypeptide. These variants are specifically covered by the term "polynucleotide sequence from" a particular valve-margin gene, such as *IND1.* In addition, the term specifically includes sequences (e.g., full length sequences) substantially identical (determined as described below) with a IND1 gene sequence and that encode proteins that retain the function of a IND1 polypeptide.

In the case of polynucleotides used to inhibit expression of an endogenous gene, the introduced sequence need not be perfectly identical to a sequence of the target endogenous gene. The introduced polynucleotide sequence will typically be at least substantially identical (as determined below) to the target endogenous sequence.

Two nucleic acid sequences or polypeptides are said to be "identical" if the sequence of nucleotides or amino acid residues respectively, in the two sequences is the same when aligned for maximum correspondence as described below. The term "complementary to" is used herein to mean that the sequence is complementary to all or a portion of a reference polynucleotide sequence.

Optimal alignment of sequences for comparison may be conducted by the local homology algorithm of Smith and Waterman Add. APL. Math. 2:482 (1981), by the homology alignment algorithm of Needle man and Wunsch J. Mol. Biol. 48:443 (1970), by the search for similarity method of Pearson and Lipman Proc. Natl. Acad. Sci. (U.S.A.) 85: 2444 (1988), by computerized implementations of these algorithms (GAP, BESTFIT, BLAST, FASTA, and TFASTA in the Wisconsin Genetics Software Package, Genetics Computer Group (GCG), 575 Science Dr., Madison, WI), or by inspection.

"Percentage of sequence identity" is determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

The term "substantial identity" of polynucleotide sequences means that a polynucleotide comprises a sequence that has at least 25% sequence identity. Alternatively, percent identity can be any integer from 25% to 100%. More preferred embodiments include at least: 25%, 30%, 35%, 40%, 45%, 50%, 55%, 60%, 65%, 70%, 75%, 80%, 85%, 90%, 95%, or 99%. compared to a reference sequence using the programs described herein; preferably BLAST using standard parameters, as described below. Accordingly, IND1 sequences of the invention include nucleic acid sequences that have substantial identity to SEQ ID NO:1. IND1 sequences of the invention also include polypeptide sequences having substantial identify to SEQ ID NO:2. One of skill will recognize that these values can be appropriately adjusted to determine corresponding identity of proteins encoded by two nucleotide sequences by taking into account codon degeneracy, amino acid similarity, reading frame positioning and the like. Substantial identity of amino acid sequences for these purposes normally means sequence identity of at least 40%. Preferred percent identity of polypeptides can be any integer from 40% to 100%. More preferred embodiments include at least 40%, 45%, 50%, 55%, 60%, 65%, 70%, 75%, 80%, 85%, 90%, 95%, or 99%. Most preferred embodiments include 61%, 62%, 63%, 64%, 65%, 66%, 67%, 68%, 69%, 70%, 71%, 72%, 73%, 74% and 75%. Polypeptides which are "substantially similar" share sequences as noted above except that residue positions which are not identical may differ by conservative amino acid changes. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, aspartic acid-glutamic acid, and asparagine-glutamine.

Another indication that nucleotide sequences are substantially identical is if two molecules hybridize to each other, or a third nucleic acid, under stringent conditions. Stringent conditions are sequence dependent and will be different in different circumstances. Generally, stringent conditions are selected to be about 5°C lower than the thermal melting point (Tm) for the specific sequence at a defined ionic strength and pH. The Tm is the temperature (under defined ionic strength and pH) at which 50% of the target sequence hybridizes to a perfectly matched probe. Typically, stringent conditions will be those in which the salt concentration is about 0.02 molar at pH 7 and the temperature is at least about 60°C.

In the present invention, mRNA encoded by IND1 genes of the invention can be identified in Northern blots under stringent conditions using cDNAs of the invention or fragments of at least about 100 nucleotides. For the purposes of this disclosure, stringent conditions for such RNA-DNA hybridizations are those which include at least one wash in 0.2X SSC at 63°C for 20 minutes, or equivalent conditions. Genomic DNA or cDNA comprising genes of the invention can be identified using the same cDNAs (or fragments of at least about 100 nucleotides) under stringent conditions, which for purposes of this disclosure, include at least one wash (usually 2) in 0.2X SSC at a temperature of at least about 50°C, usually about 55°C, for 20 minutes, or equivalent conditions.

### BRIEF DESCRIPTION OF THE DRAWINGS

Figure I illustrates the cell types of the Arabidopsis fruit at maturity.

### DETAILED DESCRIPTION

### I. Introduction

The present invention provides methods of modulating fruit development in plants. In particular, the invention provides methods of delaying or preventing fruit dehiscence by suppressing expression of an bHLH gene such as IND1 in a plant. The invention also provides transgenic plants comprising various polynucleotides encoding an bHLH polypeptide such as IND1.

The present invention relates to the previous discovery that an *agl1 aglj* double mutant plant has a delayed seed dispersal phenotype (Liljegren et al., Nature 404:766-770 (2000)). Loss-of-function mutations in the *SHP1 and SHP2* genes were produced by disruptive T-DNA insertion and homologous recombination. In the resulting *shp1 shp2* double mutant plants, the dehiscence zone failed to develop normally, and the mature fruits did not undergo dehiscence. Thus, *SHP1* or *SHP2* gene expression is required for development of the dehiscence zone. These results indicate that *SHP1* and *SHP2* regulate pod dehiscence and that manipulation of *SHP1 and SHP2* expression can allow the process of pod shatter to be controlled.

The present invention provides evidence that *IND1* is regulated by *SHP1* and *SHP2* and that expression of IND1 modulates fruit dehiscence. The present invention also provides for methods of delaying fruit dehiscence by suppressing expression of IND1.

The Arabidopsis *SHP1 and SHP2* genes encode MADS box proteins with 85% identity at the amino acid level. The *SHP1* and *SHP2* RNA expression patterns are also strikingly similar. In particular, both RNAs are specifically expressed in flowers, where they accumulate in developing carpels. In particular, strong expression of these genes is observed in the outer replum along the valve/replum boundary (Ma *et al., supra,* 1991; Savidge et al., The Plant Cell 7:721-723 (1995); Flanagan et al., The Plant Journal 10:343-353 (1996)). Thus, *SHP1* and *SHP2* are expressed in the valve margin, at least within the cells of the outer replum.

Generally, the nomenclature and the laboratory procedures in recombinant DNA technology described below are those well known and commonly employed in the art. Standard techniques are used for cloning, DNA and RNA isolation, amplification and purification. Generally enzymatic reactions involving DNA ligase, DNA polymerase, restriction endonucleases and the like are performed according to the manufacturer's specifications. These techniques and various other techniques are generally performed according to Sambrook et al., Molecular Cloning - A Laboratory Manual, 2nd. ed., Cold Spring Harbor Laboratory, Cold Spring Harbor, New York, (1989).

### II. Isolation of nucleic acids of the invention

The isolation of sequences from the genes of the invention may be accomplished by a number of techniques. For instance, oligonucleotide probes based on the sequences disclosed here can be used to identify the desired gene in a cDNA or genomic DNA library from a desired plant species. To construct genomic libraries, large segments of genomic DNA are generated by random fragmentation, e.g. using restriction endonucleases, and are ligated with vector DNA to form concatemers that can be packaged into the appropriate vector. To prepare a library of embryo-specific cDNAs such as *IND1,* mRNA is isolated from embryos and a cDNA library that contains the gene transcripts is prepared from the mRNA.

The cDNA or genomic library can then be screened using a probe based upon the sequence of a cloned IND1 gene such as the polynucleotides disclosed here. Probes may be used to hybridize with genomic DNA or cDNA sequences to isolate homologous genes in the same or different plant species.

Alternatively, the nucleic acids of interest can be amplified from nucleic acid samples using amplification techniques. For instance, polymerase chain reaction (PCR) technology to amplify the sequences of the genes directly from mRNA, from cDNA, from genomic libraries or cDNA libraries. PCR and other *in vitro* amplification methods may also be useful, for example, to clone nucleic acid sequences that code for proteins to be expressed, to make nucleic acids to use as probes for detecting the presence of the desired mRNA in samples, for nucleic acid sequencing, or for other purposes.

Appropriate primers and probes for identifying genes such as *IND1* from plant tissues are generated from comparisons of the sequences provided herein. For a general overview of PCR see PCR Protocols: A Guide to Methods and Applications. (Innis, M, Gelfand, D., Sninsky, J. and White, T., eds.), Academic Press, San Diego (1990). Appropriate primers for amplification of the genomic region of *IND1* or the *IND1* cDNA include the following primer pairs: 5'-gatgaaaatggaaaatggtatgtata-3' and 5'-gttcatcagggttgggagttgtg-3'. The amplification conditions are typically as follows. Reaction components: 10 mM Tris-HCl, pH 8.3, 50 mM potassium chloride, 1.5 mM magnesium chloride, 0.001% gelatin, 200 µM dATP, 200 µM dCTP, 200 µM dGTP, 200 µM dTTP, 0.4 µM primers, and 100 units per ml Taq polymerase. Program: 96 C for 3 min., 30 cycles of 96 C for 45 sec., 50 C for 60 sec., 72 for 60 sec, followed by 72 C for 5 min.

Polynucleotides may also be synthesized by well-known techniques as described in the technical literature. *See,* e.g., Carruthers et al., Cold Spring Harbor Symp. Quant. Biol. 47:411-418 (1982), and Adams et al., J. Am. Chem: Soc. 105:661 (1983). Double stranded DNA fragments may then be obtained either by synthesizing the complementary strand and annealing the strands together under appropriate conditions, or by adding the complementary strand using DNA polymerase with an appropriate primer sequence.

The genus of *IND1* nucleic acid sequences of the invention includes genes and gene products identified and characterized by analysis using nucleic acid sequences of the invention, including SEQ ID NO:1 and protein sequences of the invention, including SEQ ID NO:2. IND1 sequences of the invention include nucleic acid sequences having substantial identity to SEQ ID NO:1. IND1 sequences of the invention also include polypeptide sequences having substantial identity to SEQ ID NO:2.

### III. Use of nucleic acids of the invention

### A. Use of nucleic acids of the invention to inhibit or suppress gene expression

The invention provides methods of modulating fruit dehiscence in a plant by introducing into a plant a recombinant expression cassette comprising a regulatory element operably linked to a HLH polynucleotide such as *IND1.* The invention also provides methods for delaying seed dispersal in a plant by suppressing expression of a nucleic acid molecule encoding an IND1 gene product. In a transgenic plant of the invention, a nucleic acid molecule, or antisense constructs thereof, encoding an IND1 gene product can be operatively linked to an exogenous regulatory element. The invention provides, for example, a transgenic plant characterized by delayed seed dispersal having an expressed nucleic acid molecule encoding an IND1 gene product, or antisense construct thereof, that is operatively linked to an exogenous constitutive regulatory element. In one embodiment, the invention provides a transgenic plant that is characterized by delayed seed dispersal due to suppression of a nucleic acid molecule encoding an IND1 ortholog. In some preferred embodiments, suppression of IND1 expression results in reduced lignification in valve margin cells. *See also,* U.S. 6, 410, 826.

The *IND1* sequences prepared as described herein, can be used to prepare expression cassettes useful in a number of techniques, including inhibiting or suppressing expression. A number of methods can be used to inhibit gene expression in plants. For instance, antisense technology can be conveniently used. To accomplish this, a nucleic acid segment from the desired gene is cloned and operably linked to a promoter such that the antisense strand of RNA will be transcribed. The expression cassette is then transformed into plants and the antisense strand of RNA is produced. In plant cells, it has been suggested that antisense RNA inhibits gene expression by preventing the accumulation of mRNA which encodes the enzyme of interest, see, e.g., Sheehy et al., Proc. Nat. Acad. Sci. USA, 85:8805-8809 (1988); Pnueli et al., The Plant Cell 6:175-186 (1994); and Hiatt *et al.,* U.S. Patent No. 4,801,340.

The antisense nucleic acid sequence transformed into plants will be substantially identical to at least a portion of the endogenous gene or genes to be repressed. The sequence, however, does not have to be perfectly identical to inhibit expression. Thus, an antisense or sense nucleic acid molecule encoding only a portion of IND1 can be useful for producing a plant in which IND1 expression is suppressed. The vectors of the present invention can be designed such that the inhibitory effect applies to other proteins within a family of genes exhibiting homology or substantial homology to the target gene.

For antisense suppression, the introduced sequence also need not be full length relative to either the primary transcription product or fully processed mRNA. Generally, higher homology can be used to compensate for the use of a shorter sequence. Furthermore, the introduced sequence need not have the same intron or exon pattern, and homology of non-coding segments may be equally effective. Normally, a sequence of between about 30 or 40 nucleotides and about full length nucleotides should be used, though a sequence of at least about 100 nucleotides is preferred, a sequence of at least about 200 nucleotides is more preferred, and a sequence of at least about 500 nucleotides is especially preferred.

Catalytic RNA molecules or ribozymes can also be used to inhibit expression of *IND1* genes. It is possible to design ribozymes that specifically pair with virtually any target RNA and cleave the phosphodiester backbone at a specific location, thereby functionally inactivating the target RNA. In carrying out this cleavage, the ribozyme is not itself altered, and is thus capable of recycling and cleaving other molecules, making it a true enzyme. The inclusion of ribozyme sequences within antisense RNAs confers RNA-cleaving activity upon them, thereby increasing the activity of the constructs.

A number of classes of ribozymes have been identified. One class of ribozymes is derived from a number of small circular RNAs that are capable of self-cleavage and replication in plants. The RNAs replicate either alone (viroid RNAs) or with a helper virus (satellite RNAs). Examples include RNAs from avocado sunblotch viroid and the satellite RNAs from tobacco ringspot virus, lucerne transient streak virus, velvet tobacco mottle virus, solanum nodiflorum mottle virus and subterranean clover mottle virus. The design and use of target RNA-specific ribozymes is described in Haseloff et al. Nature, 334;585-591 (1988).

Another method of suppression is sense suppression (also known as co-suppression). Introduction of expression cassettes in which a nucleic acid is configured in the sense orientation with respect to the promoter has been shown to be an effective means by which to block the transcription of target genes. For an example of the use of this method to modulate expression of endogenous genes see, Napoli et al., The Plant Cell 2:279-289 (1990); Flavell, Proc. Natl. Acad. Sci., USA 91:3490-3496 (1994); Kooter and Mol, Current Opin. Biol. 4:166-171 (1993); and U.S. Patents Nos. 5,034,323, 5,231,020, and 5,283,184.

Generally, where inhibition of expression is desired, some transcription of the introduced sequence occurs. The effect may occur where the introduced sequence contains no coding sequence per se, but only intron or untranslated sequences homologous to sequences present in the primary transcript of the endogenous sequence. The introduced sequence generally will be substantially identical to the endogenous sequence intended to be repressed. This minimal identity will typically be greater than about 65%, but a higher identity might exert a more effective repression of expression of the endogenous sequences. Substantially greater identity of more than about 80% is preferred, though about 95% to absolute identity would be most preferred. As with antisense regulation, the effect should apply to any other proteins within a similar family of genes exhibiting homology or substantial homology.

For sense suppression, the introduced sequence in the expression cassette, needing less than absolute identity, also need not be full length, relative to either the primary transcription product or fully processed mRNA. This may be preferred to avoid concurrent production of some plants that are overexpressers. A higher identity in a shorter than full length sequence compensates for a longer, less identical sequence. Furthermore, the introduced sequence need not have the same intron or exon pattern, and identity of non-coding segments will be equally effective. Normally, a sequence of the size ranges noted above for antisense regulation is used.

In a preferred embodiment, expression of a nucleic acid of interest can be suppressed by the simultaneous expression of both sense and antisense constructs (Waterhouse et al., Proc. Natl. Acad. Sci. USA 95:13959-13964 (1998). *See also* Tabara et al. Science 282:430-431 (1998).

One of skill in the art will recognize that using technology based on specific nucleotide sequences (e.g., antisense or sense suppression technology), families of homologous genes can be suppressed with a single sense or antisense transcript. For instance, if a sense or antisense transcript is designed to have a sequence that is conserved among a family of genes, then multiple members of a gene family can be suppressed. Conversely, if the goal is to only suppress one member of a homologous gene family, then the sense or antisense transcript should be targeted to sequences with the most vairance between family members.

Another means of inhibiting *IND1* function in a plant is by creation of dominant negative mutations. In this approach, non-functional, mutant IND1 polypeptides, which retain the ability to interact with wild-type subunits are introduced into a plant. A dominant negative construct also can be used to suppress IND1 expression in a plant. A dominant negative construct useful in the invention generally contains a portion of the complete IND1 coding sequence sufficient, for example, for DNA-binding or for a protein-protein interaction such as a homodimeric or heterodimeric protein-protein interaction but lacking the transcriptional activity of the wild type protein. For example, a carboxy-terminal deletion mutant of AGAMOUS was used as a dominant negative construct to suppress expression of the MADS box gene AGAMOUS (Mizukami et al., Plant Cell 8:831-844 (1996)). One skilled in the art understands that, similarly, a dominant negative IND1 construct can be used to suppress IND1 expression in a plant.

### B. Use of nucleic acids of the invention to enhance gene expression

Isolated sequences prepared as described herein can also be used to prepare expression cassettes that enhance or increase endogenous *IND1* gene expression. Where overexpression of a gene is desired, the desired gene from a different species may be used to decrease potential sense suppression effects. Enhanced expression of *IND1* polynucleotides is useful, for example, to produce plants with small fruit.

Any of a number of means well known in the art can be used to increase IND 1 activity in plants. Any organ can be targeted, such as shoot vegetative organs/structures (*e.g*. leaves, stems and tubers), roots, flowers and floral organs/structures (*e.g.* bracts, sepals, petals, stamens, carpels, anthers and ovules), seed (including embryo, endosperm, and seed coat) and fruit. Alternatively, one or several IND1 genes can be expressed constitutively (*e.g*., using the CaMV 35S promoter).

One of skill will recognize that the polypeptides encoded by the genes of the invention, like other proteins, have different domains which perform different functions. Thus, the gene sequences need not be full length, so long as the desired functional domain of the protein is expressed. As explained above, IND1 polypeptides carry a bHLH domain, which is capable of binding DNA. Thus, without being bound to any particular theory or mechanism, IND1 is likely to act as a transcriptional modulator.

### C. Modification of endogenous IND1 genes

Methods for introducing genetic mutations into plant genes and selecting plants with desired traits are well known. For instance, seeds or other plant material can be treated with a mutagenic chemical substance, according to standard techniques. Such chemical substances include, but are not limited to, the following: diethyl sulfate, ethylene imine, ethyl methanesulfonate and N-nitroso-N-ethylurea. Alternatively, ionizing radiation from sources such as, X-rays or gamma rays can be used.

Modified protein chains can also be readily designed utilizing various recombinant DNA techniques well known to those skilled in the art and described for instance, in Sambrook et al., supra. Hydroxylamine can also be used to introduce single base mutations into the coding region of the gene (Sikorski, et al., (1991). Meth. Enzymol. 194: 302-318). For example, the chains can vary from the naturally occurring sequence at the primary structure level by amino acid substitutions, additions, deletions, and the like. These modifications can be used in a number of combinations to produce the final modified protein chain.

Alternatively, homologous recombination can be used to induce targeted gene modifications by specifically targeting the *IND1* gene *in vivo (see, generally,* Grewal and Klar, Genetics 146: 1221-1238 (1997) and Xu et al., Genes Dev. 10: 2411-2422 (1996)). Homologous recombination has been demonstrated in plants (Puchta et al., Experientia 50: 277-284 (1994), Swoboda et al., EMBO J. 13: 484-489 (1994); Offringa et al., Proc. Natl. Acad. Sci. USA 90: 7346-7350 (1993); and Kempin et al. Nature 389:802-803 (1997)).

In applying homologous recombination technology to the genes of the invention, mutations in selected portions of an *IND1* gene sequences (including 5' upstream, 3' downstream, and intragenic regions) such as those disclosed here are made *in vitro* and then introduced into the desired plant using standard techniques. Since the efficiency of homologous recombination is known to be dependent on the vectors used, use of dicistronic gene targeting vectors as described by Mountford et al., Proc. Natl. Acad. Sci. USA 91: 4303-4307 (1994); and Vaulont et al., Transgenic Res. 4: 247-255 (1995) are conveniently used to increase the efficiency of selecting for altered *IND1* gene expression in transgenic plants. The mutated gene will interact with the target wild-type gene in such a way that homologous recombination and targeted replacement of the wild-type gene will occur in transgenic plant cells, resulting in suppression of IND1 activity.

Alternatively, oligonucleotides composed of a contiguous stretch of RNA and DNA residues in a duplex conformation with double hairpin caps on the ends can be used. The RNA/DNA sequence is designed to align with the sequence of the target *IND1* gene and to contain the desired nucleotide change. Introduction of the chimeric oligonucleotide on an extrachromosomal T-DNA plasmid results in efficient and specific IND1 gene conversion directed by chimeric molecules in a small number of transformed plant cells. This method is described in Cole-Strauss et al., Science 273:1386-1389 (1996) and Yoon et al., Proc. Natl. Acad. Sci. USA 93: 2071-2076 (1996).

In other embodiments, the promoters derived from the IND1 genes of the invention can be used to drive expression of heterologous genes in an valve margin-specific manner. Suitable structural genes that could be used for this purpose include genes encoding cytotoxic proteins as discussed below.

Typically, desired promoters are identified by analyzing the 5' sequences of a genomic clone corresponding to the IND1 genes described here. Sequences characteristic of promoter sequences can be used to identify the promoter. Sequences controlling eukaryotic gene expression have been extensively studied. For instance, promoter sequence elements include the TATA box consensus sequence (TATAAT), which is usually 20 to 30 base pairs upstream of the transcription start site. In most instances the TATA box is required for accurate transcription initiation. In plants, further upstream from the TATA box, at positions -80 to -100, there is typically a promoter element with a series of adenines surrounding the trinucleotide G (or T) N G. J.Messing et al., in GENETIC ENGINEERING IN PLANTS, pp.221-227 (Kosage, Meredith and Hollaender, eds. (1983)).

A number of methods are known to those of skill in the art for identifying and characterizing promoter regions in plant genomic DNA (see, e.g., Jordano, et al., Plant Cell, 1: 855-866 (1989); Bustos, et al., Plant Cell, 1:839-854 (1989); Green, et al., EMBO J. 7, 4035-4044 (1988); Meier, et al., Plant Cell, 3, 309-316 (1991); and Zhang, et al., Plant Physiology 110: 1069-1079 (1996)).

### IV. Preparation of recombinant vectors

To use isolated sequences in the above techniques, recombinant DNA vectors suitable for transformation of plant cells are prepared. Techniques for transforming a wide variety of higher plant species are well known and described in the technical and scientific literature. *See*, for example, Weising et al. Ann. Rev. Genet. 22:421-477 (1988). A DNA sequence coding for the desired polypeptide, for example a cDNA sequence encoding a full length protein, will preferably be combined with transcriptional and translational initiation regulatory sequences which will direct the transcription of the sequence from the gene in the intended tissues of the transformed plant.

For example, for overexpression, a plant promoter fragment may be employed which will direct expression of the gene in all tissues of a regenerated plant. Such promoters are referred to herein as "constitutive" promoters and are active under most environmental conditions and states of development or cell differentiation. Examples of constitutive promoters include the cauliflower mosaic virus (CaMV) 35S transcription initiation region, the 1'- or 2'- promoter derived from T-DNA of Agrobacterium tumafaciens, and other transcription initiation regions from various plant genes known to those of skill.

Alternatively, the plant promoter may direct expression of the polynucleotide of the invention in a specific tissue (tissue-specific promoters) or may be otherwise under more precise environmental control (inducible promoters). Examples of tissue-specific promoters under developmental control include promoters that initiate transcription only in certain tissues, such as fruit, seeds, or flowers. As noted above, the promoters from the *IND1* genes described here are particularly useful for directing gene expression so that a desired gene product is located in the valve margin of fruit. Other suitable promoters include those from genes such as *SHP1* or *SHP2* (Savidge, B., Rounsley, S.D., and Yanofsky, M.F. (1995) Plant Cell 7: 721-733). Examples of environmental conditions that may affect transcription by inducible promoters include anaerobic conditions, elevated temperature, or the presence of light.

If proper polypeptide expression is desired, a polyadenylation region at the 3'-end of the coding region should be included. The polyadenylation region can be derived from the natural gene, from a variety of other plant genes, or from T-DNA.

The vector comprising the sequences (e.g., promoters or coding regions) from genes of the invention will typically comprise a marker gene that confers a selectable phenotype on plant cells. For example, the marker may encode biocide resistance, particularly antibiotic resistance, such as resistance to kanamycin, G418, bleomycin, hygromycin, or herbicide resistance, such as resistance to chlorosluforon or Basta.

*IND1* nucleic acid sequences of the invention are expressed recombinantly in plant cells to enhance and increase levels of endogenous IND1 polypeptides. Alternatively, antisense or other *IND1* constructs (described above) are used to suppress IND1 levels of expression. A variety of different expression constructs, such as expression cassettes and vectors suitable for transformation of plant cells can be prepared. Techniques for transforming a wide variety of higher plant species are well known and described in the technical and scientific literature. See, e.g., Weising et al. Ann. Rev. Genet. 22:421-477 (1988). A DNA sequence coding for a IND1 polypeptide, e.g., a cDNA sequence encoding a full length protein, can be combined with cis-acting (promoter) and trans-acting (enhancer) transcriptional regulatory sequences to direct the timing, tissue type and levels of transcription in the intended tissues of the transformed plant. Translational control elements can also be used.

The invention provides an *IND1* nucleic acid operably linked to a promoter which, in a preferred embodiment, is capable of driving the transcription of the IND1 coding sequence in plants. The promoter can be, e.g., derived from plant or viral sources. The promoter can be, e.g., constitutively active, inducible, or tissue specific. In construction of recombinant expression cassettes, vectors, transgenics, of the invention, a different promoters can be chosen and employed to differentially direct gene expression, e.g., in some or all tissues of a plant or animal. Typically, as discussed above, desired promoters are identified by analyzing the 5' sequences of a genomic clone corresponding to the IND1 genes described here.

### A. Constitutive Promoters

A promoter fragment can be employed which will direct expression of IND1 nucleic acid in all transformed cells or tissues, e.g. as those of a regenerated plant. The term "constitutive regulatory element" means a regulatory element that confers a level of expression upon an operatively linked nucleic molecule that is relatively independent of the cell or tissue type in which the constitutive regulatory element is expressed. A constitutive regulatory element that is expressed in a plant generally is widely expressed in a large number of cell and tissue types. Promoters that drive expression continuously under physiological conditions are referred to as "constitutive" promoters and are active under most environmental conditions and states of development or cell differentiation.

A variety of constitutive regulatory elements useful for ectopic expression in a transgenic plant are well known in the art. The cauliflower mosaic virus 35S (CaMV 35S) promoter, for example, is a well-characterized constitutive regulatory element that produces a high level of expression in all plant tissues (Odell et al., Nature 313:810-812 (1985)). The CaMV 35S promoter can be particularly useful due to its activity in numerous diverse plant species (Benfey and Chua, Science 250:959-966 (1990); Futterer et al., Physiol. Plant 79:154 (1990); Odell *et al., supra,* 1985). A tandem 35S promoter, in which the intrinsic promoter element has been duplicated, confers higher expression levels in comparison to the unmodified 35S promoter (Kay et al., Science 236:1299 (1987)). Other useful constitutive regulatory elements include, for example, the cauliflower mosaic virus 19S promoter; the Figwort mosaic virus promoter; and the nopaline synthase (nos) gene promoter (Singer et al., Plant Mol. Biol. 14:433 (1990); An, Plant Physiol. 81:86 (1986)).

Additional constitutive regulatory elements including those for efficient expression in monocots also are known in the art, for example, the pEmu promoter and promoters based on the rice Actin-1 5' region (Last et al., Theor. Appl. Genet. 81:581 (1991); Mcelroy et al., Mol. Gen. Genet. 231:150 (1991); Mcelroy et al., Plant Cell 2:163 (1990)). Chimeric regulatory elements, which combine elements from different genes, also can be useful for ectopically expressing a nucleic acid molecule encoding an IND1 polynucleotide (Comai et al., Plant Mol. Biol. 15:373 (1990)).

Other examples of constitutive promoters include the 1'- or 2'- promoter derived from T-DNA of Agrobacterium tumafaciens (see, e.g., Mengiste (1997) supra; O'Grady (1995) Plant Mol. Biol. 29:99-108); actin promoters, such as the Arabidopsis actin gene promoter (see, e.g., Huang (1997) Plant Mol. Biol. 1997 33:125-139); alcohol dehydrogenase (Adh) gene promoters (see, e.g., Millar (1996) Plant Mol. Biol. 31:897-904); *ACT11* from *Arabidopsis* (Huang et al. Plant Mol. Biol. 33:125-139 (1996)), *Cat3* from *Arabidopsis* (GenBank No. U43147, Zhong et al., Mol. Gen. Genet. 251:196-203 (1996)), the gene encoding stearoyl-acyl carrier protein desaturase from *Brassica napus* (Genbank No. X74782, Solocombe et al. Plant Physiol. 104:1167-1176 (1994)), *GPc1* from maize (GenBank No. X15596, Martinez et al. J. Mol. Biol 208:551-565 (1989)), *Gpc2* from maize (GenBank No. U45855, Manjunath et al., Plant Mol. Biol. 33:97-112 (1997)), other transcription initiation regions from various plant genes known to those of skill. *See also* Holtorf Plant Mol. Biol. 29:637-646 (1995).

### B. Inducible Promoters

Alternatively, a plant promoter may direct expression of the *IND1* nucleic acid of the invention under the influence of changing environmental conditions or developmental conditions. Examples of environmental conditions that may effect transcription by inducible promoters include anaerobic conditions, elevated temperature, drought, or the presence of light. Such promoters are referred to herein as "inducible" promoters. For example, the invention incorporates the drought-inducible promoter of maize (Busk (1997) supra); the cold, drought, and high salt inducible promoter from potato (Kirch (1997) Plant Mol. Biol. 33:897-909).

Alternatively, plant promoters which are inducible upon exposure to plant hormones, such as auxins, are used to express the nucleic acids of the invention. For example, the invention can use the auxin-response elements E1 promoter fragment (AuxREs) in the soybean (Glycine max L.) (Liu (1997) Plant Physiol. 115:397-407); the auxin-responsive Arabidopsis GST6 promoter (also responsive to salicylic acid and hydrogen peroxide) (Chen (1996) Plant J. 10: 955-966); the auxin-inducible parC promoter from tobacco (Sakai (1996) 37:906-913); a plant biotin response element (Streit (1997) Mol. Plant Microbe Interact. 10:933-93.7); and, the promoter responsive to the stress hormone abscisic acid (Sheen (1996) Science 274:1900-1902).

Plant promoters which are inducible upon exposure to chemicals reagents which can be applied to the plant, such as herbicides or antibiotics, are also used to express the nucleic acids of the invention. For example, the maize In2-2 promoter, activated by benzenesulfonamide herbicide safeners, can be used (De Veylder (1997) Plant Cell Physiol. 38:568-577); application of different herbicide safeners induces distinct gene expression patterns, including expression in the root, hydathodes, and the shoot apical meristem. *IND1* coding sequence can also be under the control of, e.g., a tetracycline-inducible promoter, e.g., as described with transgenic tobacco plants containing the Avena sativa L. (oat) arginine decarboxylase gene (Masgrau (1997) Plant J. 11:465-473); or, a salicylic acid-responsive element (Stange (1997) Plant J. 11:1315-1324; Uknes et al., Plant Cell 5:159-169 (1993); Bi et al., Plant J. 8:235-245 (1995)).

Particularly useful inducible regulatory elements include copper-inducible regulatory elements (Mett et al., Proc. Natl. Acad. Sci. USA 90:4567-4571 (1993); Furst et al., Cell 55:705-717 (1988)); tetracycline and chlor-tetracycline-inducible regulatory elements (Gatz et al., Plant J. 2:397-404 (1992); Röder et al., Mol. Gen. Genet. 243:32-38 (1994); Gatz, Meth. Cell Biol. 50:411-424 (1995)); ecdysone inducible regulatory elements (Christopherson et al., Proc. Natl. Acad. Sci. USA 89:6314-6318 (1992); Kreutzweiser et al., Ecotoxicol. Environ. Safety 28:14-24 (1994)); heat shock inducible regulatory elements (Takahashi et al., Plant Physiol. 99:383-390 (1992); Yabe et al., Plant Cell Physiol. 35:1207-1219 (1994); Ueda et al., Mol. Gen. Genet. 250:533-539 (1996)); and *lac* operon elements, which are used in combination with a constitutively expressed lac repressor to confer, for example, IPTG-inducible expression (Wilde et al., EMBO J. 11:1251-1259 (1992)). An inducible regulatory element useful in the transgenic plants of the invention also can be, for example, a nitrate-inducible promoter derived from the spinach nitrite reductase gene (Back et al., Plant Mol. Biol. 17:9 (1991)) or a light-inducible promoter, such as that associated with the small subunit of RuBP carboxylase or the LHCP gene families (Feinbaum et al., Mol. Gen. Genet. 226:449 (1991); Lam and Chua, Science 248:471 (1990)).

### C. Tissue-Specific Promoters

Alternatively, the plant promoter may direct expression of the polynucleotide of the invention in a specific tissue (tissue-specific promoters). Tissue specific promoters are transcriptional control elements that are only active in particular cells or tissues at specific times during plant development, such as in vegetative tissues or reproductive tissues. Promoters from the *IND1* genes of the invention are particularly useful for tissue-specific direction of gene expression so that a desired gene product is generated only or preferentially in embryos or seeds, as described below.

Examples of tissue-specific promoters under developmental control include promoters that initiate transcription only (or primarily only) in certain tissues, such as vegetative tissues, e.g., roots or leaves, or reproductive tissues, such as fruit, ovules, seeds, pollen, pistols, flowers, or any embryonic tissue. Reproductive tissue-specific promoters may be, e.g., ovule-specific, embryo-specific, endosperm-specific, integument-specific, seed and seed coat-specific, pollen-specific, petal-specific, sepal-specific, or some combination thereof.

The invention provides a transgenic plant that is characterized by delayed seed dispersal due to expression of a nucleic acid molecule encoding an IND1 gene product, or an antisense construct thereof, operatively linked to a dehiscence zone-selective regulatory element. The dehiscence zone-selective regulatory element can be, for example, an *SHP1* regulatory element or *SHP2* regulatory element. The *SHP1* regulatory element can be derived from the Arabidopsis *SHP1* genomic sequence disclosed herein as SEQ ID NO:5 and can be, for example, a 5' regulatory sequence or intronic regulatory element. Similarly, the *SHP2* regulatory element can be derived from the Arabidopsis *SHP2* genomic sequence disclosed herein as SEQ ID NO:6 and can be, for example, a 5' regulatory sequence or intronic regulatory element.

A dehiscence zone-selective regulatory element can be derived from a gene that is an ortholog of Arabidopsis *IND1* and is selectively expressed in the valve margin or dehiscence zone of a seed plant. A dehiscence zone-selective regulatory element can be derived, for example, from an *IND1* ortholog of the *Brassicaceae,* such as *a Brassica napus, Brassica oleracea, Brassica campestris, Brassica juncea, Brassica nigra* or *Brassica carinata IND1* ortholog. A dehiscence zone-selective regulatory element can be derived, for example, from an *IND1* canola ortholog. A dehiscence zone-selective regulatory element also can be derived, for example, from a leguminous *IND1* ortholog, such as a soybean, pea, chickpea, moth bean, broad bean, kidney bean, lima bean, lentil, cowpea, dry bean, peanut, alfalfa, lucerne, birdsfoot trefoil, clover, stylosanthes, *lotononis bainessii,* or sainfoin *IND1* ortholog.

Dehiscence zone-selective regulatory elements also can be derived from a variety of other genes that are selectively expressed in the valve margin or dehiscence zone of a seed plant. For example, the rapeseed gene RDPG1 is selectively expressed in the dehiscence zone (Petersen et al., Plant Mol. Biol. 31:517-527 (1996)). Thus, the RDPG1 promoter or an active fragment thereof can be a dehiscence zone-selective regulatory element as defined herein. Additional genes such as the rapeseed gene SAC51 also are known to be selectively expressed in the dehiscence zone; the SAC51 promoter or an active fragment thereof also can be a dehiscence zone-selective regulatory element of the invention (Coupe et al., Plant Mol. Biol. 23:1223-1232 (1993)). The skilled artisan understands that a regulatory element of any such gene selectively expressed in cells of the valve margin or dehiscence zone can be a dehiscence zone-selective regulatory element as defined herein.

Additional dehiscence zone-selective regulatory elements can be identified and isolated using routine methodology. Differential screening strategies using, for example, RNA prepared from the dehiscence zone and RNA prepared from adjacent pod material can be used to isolate cDNAs selectively expressed in cells of the dehiscence zone (Coupe *et al., supra,* 1993); subsequently, the corresponding genes are isolated using the cDNA sequence as a probe.

Enhancer trap or gene trap strategies also can be used to identify and isolate a dehiscence zone-selective regulatory element of the invention (Sundaresan *et al., supra,* 1995; Koncz et al., Proc. Natl. Acad. Sci. USA 86:8467-8471 (1989); Kertbundit et al., Proc. Natl. Acad. Sci. USA 88:5212-5216 (1991); Topping et al., Development 112:1009-1019 (1991)). Enhancer trap elements include a reporter gene such as GUS with a weak or minimal promoter, while gene trap elements lack a promoter sequence, relying on transcription from a flanking chromosomal gene for reporter gene expression. Transposable elements included in the constructs mediate fusions to endogenous loci; constructs selectively expressed in the valve margin or dehiscence zone are identified by their pattern of expression. With the inserted element as a tag, the flanking dehiscence zone-selective regulatory element is cloned using, for example, inverse polymerase chain reaction methodology (see, for example, Aarts et al., Nature 363:715-717 (1993); *see also,* Ochman *et al.,* "Amplification of Flanking Sequences by Inverse PCR," in Innis *et al.,* supra, 1990). The Ac/Ds transposition system of Sundaresan et al., Genes. Devel. 9:1797-1810 (1995), can be particularly useful in identifying and isolating a dehiscence zone-selective regulatory element of the invention.

Dehiscence zone-selective regulatory elements also can be isolated by inserting a library of random genomic DNA fragments in front of a promoterless reporter gene and screening transgenic plants transformed with the library for dehiscence zone-selective reporter gene expression. The promoterless vector pROA97, which contains the npt gene and the GUS gene each under the control of the minimal 35S promoter, can be useful for such screening. The genomic library can be, for example, Sau3A fragments of Arabidopsis thaliana genomic DNA or genomic DNA from, for example, another Brassicaceae of interest (Ott et al., Mol. Gen. Genet. 223:169-179 (1990); Claes et al., The Plant Journal 1:15-26 (1991)).

Dehiscence zone-selective expression of a regulatory element of the invention can be demonstrated or confirmed by routine techniques, for example, using a reporter gene and in situ expression analysis. The GUS and firefly luciferase reporters are particularly useful for in situ localization of plant gene expression (Jefierson et al., EMBO J. 6:3901 (1987); Ow et al., Science 334:856 (1986)), and promoterless vectors containing the GUS expression cassette are commercially available, for example, from Clontech (Palo Alto, CA). To identify a dehiscence zone-selective regulatory element of interest such as an *IND1* regulatory element, one or more nucleotide portions of the *IND1* gene can be generated using enzymatic or PCR-based methodology (Glick and Thompson, *supra,* 1993; Innis *et al., supra,* 1990); the resulting segments are fused to a reporter gene such as GUS and analyzed as described above.

Other tissue-specific promoters include seed promoters. Suitable seed-specific promoters are derived from the following genes: *MAC1* from maize (Sheridan (1996) Genetics 142:1009-1020); *Cat3* from maize (GenBank No. L05934, Abler (1993) Plant Mol. Biol. 22:10131-1038); *vivparous-1* from Arabidopsis (Genbank No. U93215); *atmyc1* from Arabidopsis (Urao (1996) Plant Mol. Biol. 32:571-57; Conceicao (1994) Plant 5:493-505); *napA* from Brassica napus (GenBank No. J02798, Josefsson (1987) JBL 26:12196-1301); and the napin gene family from Brassica napus (Sjodahl (1995) Planta 197:264-271).

A variety of promoters specifically active in vegetative tissues, such as leaves, stems, roots and tubers, can also be used to express the *IND1* nucleic acids of the invention. For example, promoters controlling patatin, the major storage protein of the potato tuber, can be used, see, e.g., Kim (1994) Plant Mol. Biol. 26:603-615; Martin (1997) Plant J. 11:53-62. The ORF13 promoter from Agrobacterium rhizogenes which exhibits high activity in roots can also be used (Hansen (1997) Mol. Gen. Genet. 254:337-343. Other useful vegetative tissue-specific promoters include: the tarin promoter of the gene encoding a globulin from a major taro (Colocasia esculenta L. Schott) corm protein family, tarin (Bezerra (1995) Plant Mol. Biol. 28:137-144); the curculin promoter active during taro corm development (de Castro (1992) Plant Cell 4:1549-1559) and the promoter for the tobacco root-specific gene TobRB7, whose expression is localized to root meristem and immature central cylinder regions (Yamamoto (1991) Plant Cell 3:371-382).

Leaf-specific promoters, such as the ribulose biphosphate carboxylase (RBCS) promoters can be used. For example, the tomato RBCS1, RBCS2 and RBCS3A genes are expressed in leaves and light-grown seedlings, only RBCS 1 and RBCS2 are expressed in developing tomato fruits (Meier (1997) FEBS Lett. 415:91-95). A ribulose bisphosphate carboxylase promoters expressed almost exclusively in mesophyll cells in leaf blades and leaf sheaths at high levels, described by Matsuoka (1994) Plant J. 6:311-319, can be used. Another leaf-specific promoter is the light harvesting chlorophyll a/b binding protein gene promoter, see, e.g., Shiina (1997) Plant Physiol. 115:477-483; Casal (1998) Plant Physiol. 116:1533-1538. The Arabidopsis thaliana myb-related gene promoter (Atmyb5) described by Li (1996) FEBS Lett. 379:117-121, is leaf-specific. The Atmyb5 promoter is expressed in developing leaf trichomes, stipules, and epidermal cells on the margins of young rosette and cauline leaves, and in immature seeds. Atmyb5 mRNA appears between fertilization and the 16 cell stage of embryo development and persists beyond the heart stage. A leaf promoter identified in maize by Busk (1997) Plant J. 11: 1285-1295, can also be used.

Another class of useful vegetative tissue-specific promoters are meristematic (root tip and shoot apex) promoters. For example, the *"SHOOTMERISTEMLESS"* and *"SCARECROW"* promoters, which are active in the developing shoot or root apical meristems, described by Di Laurenzio (1996) Cell 86:423-433; and, Long (1996) Nature 379:66-69; can be used. Another useful promoter is that which controls the expression of 3-hydroxy-3- methylglutaryl coenzyme A reductase HMG2 gene, whose expression is restricted to meristematic and floral (secretory zone of the stigma, mature pollen grains, gynoecium vascular tissue, and fertilized ovules) tissues (see, e.g., Enjuto (1995) Plant Cell. 7:517-527). Also useful are kn1-related genes from maize and other species which show meristem-specific expression, see, e.g., Granger (1996) Plant Mol. Biol. 31:373-378; Kerstetter (1994) Plant Cell 6:1877-1887; Hake (1995) Philos. Trans. R. Soc. Lond. B. Biol. Sci. 350:45-51. For example, the *Arabidopsis thaliana* KNAT1 promoter. In the shoot apex, KNAT1 transcript is localized primarily to the shoot apical meristem; the expression of KNAT1 in the shoot meristem decreases during the floral transition and is restricted to the cortex of the inflorescence stem (see, e.g., Lincoln (1994) Plant Cell 6:1859-1876).

One of skill will recognize that a tissue-specific promoter may drive expression of operably linked sequences in tissues other than the target tissue. Thus, as used herein a tissue-specific promoter is one that drives expression preferentially in the target tissue, but may also lead to some expression in other tissues as well.

In another embodiment, a *IND1* nucleic acid is expressed through a transposable element. This allows for constitutive, yet periodic and infrequent expression of the constitutively active polypeptide. The invention also provides for use of tissue-specific promoters derived from viruses which can include, e.g., the tobamovirus subgenomic promoter (Kumagai (1995) Proc. Natl. Acad. Sci. USA 92:1679-1683; the rice tungro bacilliform virus (RTBV), which replicates only in phloem cells in infected rice plants, with its promoter which drives strong phloem-specific reporter gene expression; the cassava vein mosaic virus (CVMV) promoter, with highest activity in vascular elements, in leaf mesophyll cells, and in root tips (Verdaguer (1996) Plant Mol. Biol. 31:1129-1139).

### V. Production of transgenic plants

DNA constructs of the invention may be introduced into the genome of the desired plant host by a variety of conventional techniques. For example, the DNA construct may be introduced directly into the genomic DNA of the plant cell using techniques such as electroporation and microinjection of plant cell protoplasts, or the DNA constructs can be introduced directly to plant tissue using ballistic methods, such as DNA particle bombardment. Alternatively, the DNA constructs may be combined with suitable T-DNA flanking regions and introduced into a conventional Agrobacterium tumefaciens host vector. The virulence functions of the *Agrobacterium tumefaciens* host will direct the insertion of the construct and adjacent marker into the plant cell DNA when the cell is infected by the bacteria.

Microinjection techniques are known in the art and well described in the scientific and patent literature. The introduction of DNA constructs using polyethylene glycol precipitation is described in Paszkowski et al. Embo J. 3:2717-2722 (1984). Electroporation techniques are described in Fromm et al. Proc. Natl. Acad. Sci. USA 82:5824 (1985). Ballistic transformation techniques are described in Klein et al. Nature 327:70-73 (1987).

*Agrobacterium tumefaciens-*mediated transformation techniques, including disarming and use of binary vectors, are well described in the scientific literature. See, for example Horsch et al. Science 233:496-498 (1984), and Fraley et al. Proc. Natl. Acad. Sci. USA 80:4803 (1983).

Transformed plant cells which are derived by any of the above transformation techniques can be cultured to regenerate a whole plant which possesses the transformed genotype and thus the desired phenotype such as seedlessness. Such regeneration techniques rely on manipulation of certain phytohormones in a tissue culture growth medium, typically relying on a biocide and/or herbicide marker which has been introduced together with the desired nucleotide sequences. Plant regeneration from cultured protoplasts is described in Evans et al., Protoplasts Isolation and Culture, Handbook of Plant Cell Culture, pp. 124-176, MacMillilan Publishing Company, New York, 1983; and Binding, Regeneration of Plants, Plant Protoplasts, pp. 21-73, CRC Press, Boca Raton, 1985. Regeneration can also be obtained from plant callus, explants, organs, or parts thereof. Such regeneration techniques are described generally in Klee et al. Ann. Rev. of Plant Phys. 38:467-486 (1987).

The nucleic acids of the invention can be used to confer desired traits on essentially any plant. Thus, the invention has use over a broad range of plants, including species from the genera Asparagus, Atropa, Avena, Brassica, Citrus, Citrullus, Capsicum, Cucumis, Cucurbita, Daucus, Fragaria, Glycine, Gossypium, Helianthus, Heterocallis, Hordeum, Hyoscyamus, Lactuca, Linum, Lolium, Lycopersicon, Malus, Manihot, Majorana, Medicago, Nicotiana, Oryza, Panieum, Pannesetum, Persea, Pisum, Pyrus, Prunus, Raphanus, Secale, Senecio, Sinapis, Solanum, Sorghum, Trigonella, Triticum, Vitis, Vigna, and, Zea. A useful plant of the invention can be a dehiscent seed plant, and a particularly useful plant of the invention can be a member of the *Brassicaceae,* such as rapeseed, or a member of the *Fabaceae,* such as a soybean, pea, lentil or bean plant.

In one embodiment, the invention provides a dehiscent seed plant that is characterized by delayed seed dispersal due to suppressed expression of a nucleic acid molecule encoding an IND1 gene product in the dehiscent seed plant. As used herein, the term "dehiscent seed plant" means a plant that produces a dry dehiscent fruit, which has fruit walls that open to permit escape of the seeds contained therein. Dehiscent fruits commonly contain several seeds and include the fruits known, for example, as legumes, capsules and siliques.

In one embodiment, the invention provides a plant that is characterized by delayed seed dispersal due to suppressed expression of a nucleic acid molecule encoding an IND1 gene product, where the plant is a member of the *Brassicaceae.* The *Brassicaceae,* commonly known as the Brassicas, are a diverse group of crop plants with great economic value worldwide (see, for example, Williams and Hill, Science 232:1385-1389 (1986)). The *Brassicaceae* produce seed oils for margarine, salad oil, cooking oil, plastic and industrial uses; condiment mustard; leafy, stored, processed and pickled vegetables; animal fodders and green manures for soil rejuvenation. A particularly useful non-naturally occurring Brassica plant of the invention is the oilseed plant canola.

There are six major Brassica species of economic importance, each containing a range of plant forms. *Brassica napus* includes plants such as the oilseed rapes and rutabaga. *Brassica oleracea* are the cole crops such as cabbage, cauliflower, kale, kohlrabi and Brussels sprouts. *Brassica campestris (Brassica rapa)* includes:plants such as Chinese cabbage, turnip and pak choi. *Brassica juncea* includes a variety of mustards; *Brassica nigra* is the black mustard; and *Brassica carinata* is Ethiopian mustard. The skilled artisan understands that any member of the *Brassicaceae* can be modified as disclosed herein to produce a non-naturally occurring Brassica plant characterized by delayed seed dispersal.

In a second embodiment, the invention provides a plant that is characterized by delayed seed dispersal due to suppressed expression of a nucleic acid molecule encoding an IND1 gene product, where the plant is a member of the *Fabaceae.* The *Fabaceae,* which are commonly known as members of the pea family, are plants that produce a characteristic dry dehiscent fruit known as a legume. The legume is derived from a single carpel and dehisces along the suture of the carpel margins and along the median vein. The *Fabaceae* encompass both grain legumes and forage legumes. Grain legumes include, for example, soybean (glycine), pea, chickpea, moth bean, broad bean, kidney bean, lima bean, lentil, cowpea, dry bean and peanut. Forage legumes include alfalfa, lucerne, birdsfoot trefoil, clover, stylosanthes species, *lotononis bainessii* and sainfoin. The skilled artisan will recognize that any member of the *Fabaceae* can be modified as disclosed herein to produce a non-naturally occurring plant of the invention characterized by delayed seed dispersal.

A non-naturally occurring plant of the invention characterized by delayed seed dispersal also can be a member of the plant genus *Cuphea* (family *Lythraceae).* A *Cuphea* plant is particularly valuable since *Cuphea* oilseeds contain industrially and nutritionally important medium-chain fatty acids, especially lauric acid, which is currently supplied only by coconut and palm kernel oils.

A non-naturally occurring plant of the invention also can be, for example, one of the monocotyledonous grasses, which produce many of the valuable small-grain cereal crops of the world. Suppression of IND1 expression as described above, can be useful in generating a non-naturally occurring small grain cereal plant, such as a barley, wheat, oat, rye, orchard grass, guinea grass, sorghum or turf grass plant characterized by delayed seed dispersal.

### VI. Additional modifications that modulate seed dispersal

It should be recognized that a plant of the invention, which contains an exogenous *IND1* polynucleotide, also can contain one or more additional modifications, including naturally and non-naturally occurring modifications, that can modulate the delay in seed dispersal. For example, the plant hormone ethylene promotes fruit dehiscence, and modified expression or activity of positive or negative regulators of the ethylene response can be included in a plant of the invention (see, generally, Meakin and Roberts, J. Exp. Botany 41:1003-1011 (1990); Ecker, Science 268:667-675 (1995); Chao et al., Cell 89:1133-1144 (1997)).

Mutations in positive regulators of the ethylene response show a reduction or absence of responsiveness to treatment with exogenous ethylene. Arabidopsis mutations in positive regulators of the ethylene response include mutations in *etr,* which inactivate a histidine kinase ethylene receptor (Bleeker et al., Science 241:1086-1089 (1988); Schaller and Bleeker, Science 270:1809-1811 (1995)); *ers* (Hua et al., Science 269:1712-1714 (1995)); *ein2* (Guzman and Ecker, Plant Cell 2:513 (1990)); *ein3* (Rothenberg and Ecker, Sem. Dev. Biol. Plant Dev. Genet. 4:3-13 (1993); Kieber and Ecker, Trends Genet. 9:356-362 (1993)); *ain1* (van der Straeten et al., Plant Physiol. 102:401-408 (1993)); *eti* (Harpham et al., An. Bot. 68:55 (1991)) and *ein4, ein5, ein6,* and *ein7* (Roman et al., Genetics 139: 1393-1409 (1995)). Similar genetic functions are found in other plant species; for example, the never-ripe mutation corresponds to *etr* and confers ethylene insensitivity in tomato (Lanahan et al., The Plant Cell 6:521-530 (1994); Wilkinson et al., Science 270:1807-1809 (1995)). A plant of the invention can include a modification that results in altered expression or activity of any such positive regulator of the ethylene response. A mutation in a positive regulator, for example, can be included in a plant of the invention and can modify the delay in seed dispersal in such plants, for example, by further postponing the delay in seed dispersal.

Mutations in negative regulators of the ethylene response display ethylene responsiveness in the absence of exogenous ethylene. Such mutations include those relating to ethylene overproduction, for example, the eto1, eto2, and eto3 mutants, and those relating to constitutive activation of the ethylene signalling pathway, for example, mutations in CTR1, a negative regulator with sequence similarity to the Raf family of protein kinases (Kieber et al., Cell 72:427-441 (1993)).

A plant of the invention can include a modification that results in altered expression or activity of any such negative regulator of the ethylene response. A mutation resulting in ethylene responsiveness in the absence of exogenous ethylene, for example, can be included in a non-naturally occurring plant of the invention and can modify, for example, diminish, the delay in seed dispersal.

Fruit morphological mutations also can be included in a plant of the invention. Such mutations include those in carpel identity genes such as *AGAMOUS* (Bowman *et al., supra,* 1989; Yanofsky *et al., supra,* 1990) and in genes required for normal fruit development such as *ETTIN, CRABS CLAW, SPATULA, AGL8* and TOUSLED (Sessions et al., Development 121:1519-1532 (1995); Alvarez and Smyth, Flowering Newsletter 23:12-17 (1997); and Roe et al., Cell 75:939-950 (1993)). Thus, it is understood that a plant of the invention can include one or more additional genetic modifications, which can diminish or enhance the delay in seed dispersal.

### VII. Expression of cytotoxic gene products

The present invention also provides a recombinant nucleic acid molecule that includes a dehiscence zone-selective regulatory element operatively linked to a nucleic acid molecule encoding a cytotoxic gene product. Further provided herein is a plant of the invention that is characterized by delayed seed dispersal due to expression of a recombinant nucleic acid molecule having a dehiscence zone--selective regulatory element operatively linked to a nucleic acid molecule encoding a cytotoxic gene product.

A cytotoxic gene product is a gene product that causes the death of the cell in which it is expressed and, preferably, does not result in the death of cells other than the cell in which it is expressed. Thus, expression of a cytotoxic gene product from a dehiscence zone-selective regulatory element can be used to ablate the dehiscence zone without disturbing neighboring cells of the replum or valve. A variety of cytotoxic gene products useful in seed plants are known in the art including, for example, diphtheria toxin A chain polypeptides; RNase T1; Barnase RNase; ricin toxin A chain polypeptides; and herpes simplex virus thymidine kinase (tk) gene products. While the diphtheria toxin A chain, RNase T1 and Barnase RNase are preferred cytotoxic gene products, the skilled person recognizes that these, or other cytotoxic gene products can be used with a dehiscence zone-selective regulatory element to generate a non-naturally occurring seed plant characterized by delayed seed dispersal.

Diphtheria toxin is the naturally occurring toxin of Comebacterium diphtheriae, which catalyzes the ADP-ribosylation of elongation factor 2, resulting in inhibition of protein synthesis and consequent cell death (Collier, Bacteriol. Rev. 39:54-85 (1975)). A single molecule of the fully active toxin is sufficient to kill a cell (Yamaizumi et al., Cell 15:245-250 (1978)). Diphtheria toxin has two subunits: the diphtheria toxin B chain directs internalization to most eukaryotic cells through a specific membrane receptor, whereas the A chain encodes the toxic catalytic domain. The catalytic DT-A chain does not include a signal peptide and is not secreted. Further, any DT-A released from dead cells in the absence of the diphtheria toxin B chain is precluded from cell attachment. Thus, DT-A is cell autonomous and directs killing only of the cells in which it is expressed without apparent damage to neighboring cells. The DT-A expression cassette of Palmiter et al., which contains the 193 residues of the A chain engineered with a synthetic ATG and lacking the native leader sequence, is particularly useful in the seed plants of the invention (Palmiter et al., Cell 50:435-443 (1987); Greenfield et al., Proc. Natl. Acad. Sci., USA 80:6853-6857 (1983)).

RNase T1 of Aspergillus oryzae and Barnase RNase of Bacillus amylolique-faciens also are cytotoxic gene products useful in the seed plants of the invention (Thorsness and Nasrallah, Methods in Cell Biology 50:439-448 (1995)). Barnase RNase may be more generally toxic to plants than RNase T1 and, thus, is preferred in the methods of the invention.

Ricin, a ribosome-inactivating protein produced by castor bean seeds, also is a cytotoxic gene product useful in a non-naturally occurring seed plant of the invention. The ricin toxin A chain polypeptide can be used to direct cell-specific ablation as described, for example, in Moffat et al., Development 114:681-687 (1992). Plant ribosomes are variably susceptible to the plant-derived ricin toxin. The skilled person understands that the toxicity of ricin depends is variable and should be assessed for toxicity in the seed plant species of interest (see Olsnes and Pihl, Molecular Action of Toxins and Viruses, pages 51-105, Amsterdam: Elsevier Biomedical Press (1982)).

### EXAMPLES

The following examples are offered to illustrate, but no to limit the claimed invention.

### Example 1:

The GT140 valve margin marker (Sundaresan, V., et al. Genes Dev. 9, 1797-1810 (1995)) is expressed at the valve margin of the developing gynoecium just prior to fertilization (stage 13) and this pattern persists in the mature fruit (stage 17). As expression of this marker is largely absent from the valve margins of *shp1 shp2* indehiscent fruits (Liljegren, S.J., Ditta, G.S., Eshed, Y., Savidge, B., Bowman, J.L., and Yanofsky, M.F. Nature, in press), it was expected that the gene corresponding to this marker might also be involved in valve margin development and be required for fruit dehiscence.

To isolate flanking genomic sequence from the GT140 marker insertion site, TAIL/PCR was performed as previously described (Tsugeki, R., et al. Plant J. 10, 479-489 (1996)). Subsequent sequencing of the isolated PCR products demonstrated that they correspond to a fully sequenced BAC from chromosome 4, available in the public database as part of the Arabidopsis Genome Initiative. The GT140 insertion is located between two genes, one encoding a predicted basic helix-loop-helix (bHLH) transcription factor and the other representing a novel gene.

Through several lines of subsequent investigation, it was confirmed that the bHLH transcription factor (herein referred to as *IND1* as noted below) was the relevant gene (SEQ ID NO:1). Promoter/enhancer::GUS fusions of the *IND1* gene were introduced into wild-type plants and found to express GUS in an identical pattern to that of the GT140 marker line. Interestingly, approximately 25% of the transgenic lines failed to express significant GUS activity and displayed an indehiscent phenotype. The most likely explanation of these results is that the INDI::GUS fusions, as well as of the endogenous *IND1* gene, were cosuppressed. Subsequent RNA blotting confirmed a down regulation of the *IND1* gene in these lines, and further RNA blotting showed, as expected, a decrease in *IND1* gene expression in *shp1 shp2* fruits.

In parallel to the studies of the GT140 valve margin marker described above, screens for Arabidopsis mutants producing indehiscent fruits were also carried out. Besides obtaining additional alleles of *SHP1 and SHP2* through EMS mutagenesis of *shp2-1* and *shp1-1* seed stocks, indehiscent mutants that were not allelic to either *SHP1* or *SHP2,* respectively were also obtained. Because the GT140 studies suggested the possibility that one or more of these indehiscent mutants might correspond to the *IND1* gene, *IND1* from several of these mutants was cloned and sequenced. Four alleles represent independent mutant alleles of *IND1.* The strongest allele, *ind1-2*, contains a single nucleotide deletion within codon 55 that results in a frameshift and production of a truncated protein of 64 rather than 198 amino acids. The *ind1-1* and *ind1-3* alleles contain nucleotide substitutions at codons 141 and 128 that changes a leucine amino acid to a phenylalanine and an arginine to a histidine, respectively. These affected amino acids are both at conserved positions within the bHLH domain. The *ind1-4* allele contains a nucleotide substitution at codon 92 that changes a glutamine to a stop codon, causing production of a truncated protein of 91 amino acids. Since inactivation of this bHLH transcription factor prevents fruit dehiscence, the gene is referred to as *INDEHISCENTI (IND1)* and the mutant as, *ind1.* To date, *ind1* represents the only reported single gene mutation in Arabidopsis that specifically blocks fruit dehiscence.

### SEQUENCE LISTING

SEQ ID NO:1 IND1 genomic
   Sequence Range: 1 to 3856
SEQ ID NO:2 IND1 protein
SEQ ID NO:3 IND1 5' promoter
SEQ ID NO:4 IND1 3' promoter
SEQ ID NO:5 SHP1 genomic
SEQ ID NO:6 SHP2 genomic

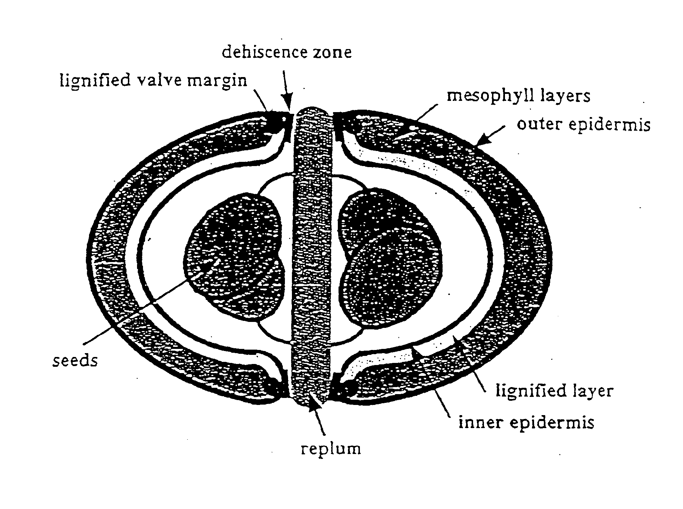

## Claims

1. An expression cassette comprising a promoter operably linked to an IND1 polynucleotide sequence, or a complement thereof, encoding an IND1 polypeptide at least about 70% identical to SEQ ID NO: 2.

2. The expression cassette of claim 1 wherein the IND1 polypeptide comprises SEQ ID NO: 2.

3. The expression cassette of claim 1 wherein the IND1 polynucleotide comprises positions from about 2765 to about 3361 of SEQ ID NO: 1.

4. The expression cassette of claim 3 wherein the IND1 polynucleotide comprises SEQ ID NO: 1.

5. An expression cassette comprising a heterologous promoter operably linked to a polynucleotide, or a complement thereof, wherein the polynucleotide is at least 60% identical to at least 200 contiguous nucleotides of a sequence encoding SEQ ID NO: 2.

6. The expression cassette of claim 5 wherein the sequence is SEQ ID NO: 1.

7. The expression cassette of claim 5 wherein the polynucleotide is at least 60% identical to a nucleotide sequence encoding SEQ ID NO: 2.

8. The expression cassette of any one of claims 1 to 7 wherein the promoter is constitutive.

9. The expression cassette of any one of claims 1 to 7 wherein the promoter is tissue specific.

10. The expression cassette of claim 9 wherein the promoter is a dehiscence zone specific promoter.

11. The expression cassette of claim 10 wherein the promoter comprises positions from about 1 to about 2764 or from about 3362 to about 3856 of SEQ ID NO: 1.

12. A plant comprising a recombinant expression cassette comprising a promoter operably linked to an IND1 polynucleotide sequence encoding an IND1 polypeptide at least about 70% identical to SEQ ID NO: 2.

13. A plant comprising a recombinant expression cassette as defined in claim 5.

14. A method of delaying fruit dehiscence in a plant, the method comprising suppressing expression of an IND1 nucleic acid in the plant by introducing into the plant a recombinant expression cassette comprising a promoter operably linked to a polynucleotide sequence encoding an IND1 polypeptide at least about 70% identical to SEQ ID NO: 2.

15. The plant of claim 12 or 13 wherein the polynucleotide sequence is operably linked to the promoter in the antisense orientation.

16. The method of claim 14 wherein the polynucleotide sequence is operably linked to the promoter in the antisense orientation

17. The plant of claim 12 or 13 wherein the polynucleotide sequence is operably linked to the promoter in the sense orientation.

18. The method of claim 14 wherein the polynucleotide sequence is operably linked to the promoter in the sense orientation.

19. The plant of claim 17, when dependent on claim 12, wherein the polynucleotide sequence further comprises a second polynucleotide sequence encoding the IND1 polypeptide wherein the second polynucleotide sequence is operably linked to a second promoter in the antisense orientation.

20. The method of claim 18, wherein the polynucleotide sequence further comprises a second polynucleotide sequence encoding the IND1 polypeptide wherein the second polynucleotide sequence is operably linked to a second promoter in the antisense orientation

21. The plant of claim 17 when dependent on claim 13 wherein the plant further comprises a second polynucleotide at least 60% identical to at least 200 contiguous nucleotides of a sequence encoding SEQ ID NO: 2, wherein the second polynucleotide sequence is operably linked to a second promoter in the antisense orientation.

22. The plant of claim 12 or 13 wherein lignification is reduced in valve margin cells.

23. The method of claim 14 wherein lignification is reduced in valve margin cells.

24. The plant of claim 12 wherein the promoter is as defined in claim 10 or claim 11.

25. The method of claim 14 wherein the promoter is as defined in claim 10 or claim 11.

26. The plant of claim 13 wherein the promoter is as defined in claim 8 or 10.

27. The method of claim 14 wherein the recombinant expression cassette is introduced into the plant using *Agrobacterium.*

28. The method of claim 14 wherein the IND1 polypeptide comprises SEQ ID NO: 2.

29. The method of claim 14 wherein the IND1 polynucleotide comprises positions from about 2765 to about 3361 of SEQ ID NO: 1.

30. The method of claim 14 wherein the IND1 polynucleotide comprises SEQ ID NO: 1.

31. The plant of claim 13 wherein the sequence is SEQ ID NO: 1.

32. The plant of claim 13 wherein the polynucleotide is at least 60% identical to a nucleotide sequence encoding SEQ ID NO: 2.

33. The plant of claim 13 wherein the plant is **characterised by** delayed seed dehiscence compared to a plant not comprising the expression cassette.

34. A method of delaying fruit dehiscence in a plant, the method comprising:
(i) suppressing the expression of an IND1 gene or a polypeptide at least 60% identical to SEQ ID NO:2 in the plant by introducing into the plant a recombinant expression cassette comprising a polynucleotide sequence at least about 70% identical to positions from about 1 to about 2764 or from about 3362 to about 3586 of SEQ ID NO: 1; and
(ii) selecting a plant with delayed fruit dehiscence compared to a plant in which expression of the IND1 gene or the polypeptide is not suppressed.

35. The method of claim 34, part (i) wherein the polynucleotide sequence comprises positions from about 1 to about 2764 or from about 3362 to about 3856 of SEQ ID NO: 1.

36. The method of claim 34 wherein lignification is reduced in valve margin cells.

37. A method for delaying fruit dehiscence in a plant, the method comprising the steps of:
(i) treating seeds or plant material with a mutagenic chemical substance or with ionising radiation;
(ii) identifying plants with a mutated *IND1* gene, wherein the *IND1* gene, prior to being mutated, encodes a polypeptide having at least 60% sequence identity to SEQ ID NO:2; and
(iii) selecting a plant with delayed fruit dehiscence compared to a plant in which the *IND1* gene is not mutated.

38. The method of claim 34, part (i) wherein the suppressing step comprises introducing into the plant a recombinant expression cassette as defined in any one of claims 5 to 7.

39. The method of claim 38 wherein the polynucleotide is operably linked to the promoter in the antisense orientation.

40. The method of claim 38 wherein the polynucleotide is operably linked to the promoter in the sense orientation.

41. The method of claim 40 wherein the polynucleotide further comprises a second polynucleotide at least 60% identical to at least 200 contiguous nucleotides of a sequence encoding SEQ ID NO: 2, wherein the second polylnucleotide is operably linked to a second promoter in the antisense orientation.

42. The method of claim 38 wherein the promoter is a dehiscence zone-selective regulatory element.

43. The method of claim 38 wherein the recombinant expression cassette is introduced into the plant using *Agrobacterium.*

44. A plant comprising a modified polynucleotide at least 65% identical to SEQ ID NO: 1, wherein the plant displays delayed fruit dehiscence compared to a plant lacking the modified polynucleotide.

45. The plant of claim 44 wherein the modified polynucleotide is at least 70% identical to SEO ID NO: 1.

46. A method of creating a plant with delayed fruit dehiscence, the method comprising introducing into said plant a recombinant expression cassette comprising a promoter operably linked to a polynucleotide that is at least 60% identical to at least 200 contiguous nucleotides of SEQ ID NO: 1 and selecting a plant with delayed fruit dehiscence compared to a plant lacking the recombinant expression cassette.

47. The method of claim 46 comprising expressing in the plant a sense polynucleotide at least 65% identical to at least 200 contiguous nucleotides of SEQ ID NO: 1 and expressing in the plant an antisense polynucleotide at least 65% identical to at least 200 contiguous nucleotides of SEQ ID NO: 1.

## Patentansprüche

1. Expressionskassette, umfassend einen Promotor, der operabel an eine IND1-Polynucleotidsequenz gebunden ist, oder ein Komplement davon, kodierend für ein IND1-Polypeptid, das eine Identität von zumindest 70 % mit Seq.-ID Nr. 2 aufweist.

2. Expressionskassette nach Anspruch 1, worin das IND1-Polypeptid Seq.-ID Nr. 2 umfasst.

3. Expressionskassette nach Anspruch 1, worin das IND1-Polynucleotid Positionen von etwa 2765 bis etwa 3361 aus Seq.-ID Nr. 1 umfasst.

4. Expressionskassette nach Anspruch 3, worin das IND1-Polynucleotid Seq.-ID Nr. 1 umfasst.

5. Expressionskassette, umfassend einen heterologen Promotor, der operabel an ein Polynucleotid gebunden ist, oder ein Komplement davon, worin das Polynucleotid eine Identität von zumindest 60 % mit zumindest 200 zusammenhängenden Nucleotiden einer Sequenz aufweist, die für Seq.-ID Nr. 2 kodiert.

6. Expressionskassette nach Anspruch 5, worin es sich bei der Sequenz um Seq.-ID Nr. 1 handelt.

7. Expressionskassette nach Anspruch 5, worin das Polynucleotid eine Identität von zumindest 60 % mit einer Nucleotidsequenz aufweist, die für Seq.-ID Nr. 2 kodiert.

8. Expressionskassette nach einem der Ansprüche 1 bis 7, worin der Promotor konstitutiv ist.

9. Expressionskassette nach einem der Ansprüche 1 bis 7, worin der Promotor gewebespezifisch ist.

10. Expressionskassette nach Anspruch 9, worin der Promotor ein Promotor ist, der für eine Dehiszenzzone spezifisch ist.

11. Expressionskassette nach Anspruch 10, worin der Promotor Positionen von etwa 1 bis etwa 2764 oder von etwa 3362 bis etwa 3856 aus Seq.-ID Nr. 1 umfasst.

12. Pflanze, umfassend eine rekombinante Expressionskassette, die einen Promotor umfasst, der operabel an eine IND1-Polynucleotidsequenz gebunden ist, die für ein IND1-Polypeptid kodiert, das eine Identität von zumindest etwa 70 % mit Seq.-ID Nr. 2 aufweist.

13. Pflanze, umfassend eine rekombinante Expressionskassette wie in Anspruch 5 definiert.

14. Verfahren zur Verzögerung der Frucht-Dehiszenz bei einer Pflanze, wobei das Verfahren die Suppression der Expression einer IND1-Nucleinsäure in der Pflanze durch das Einführen einer rekombinanten Expressionskassette, die einen Promotor umfasst, der operabel an eine Polynucleotidsequenz gebunden ist, die für ein IND1-Polypeptid kodiert, das eine Identität von zumindest 70 % mit Seq.-ID Nr. 2 aufweist, in die Pflanze umfasst.

15. Pflanze nach Anspruch 12 oder 13, worin die Polynucleotidsequenz in der Antisense-Ausrichtung operabel an den Promotor gebunden ist.

16. Verfahren nach Anspruch 14, worin die Polynucleotidsequenz in der Antisense-Ausrichtung operabel an den Promotor gebunden ist.

17. Pflanze nach Anspruch 12 oder 13, worin die Polynucleotidsequenz in der Sense-Ausrichtung operabel an den Promotor gebunden ist.

18. Verfahren nach Anspruch 14, worin die Polynucleotidsequenz in der Sense-Ausrichtung operabel an den Promotor gebunden ist.

19. Pflanze nach Anspruch 17, bei Abhängigkeit von Anspruch 12, worin die Polynucleotidsequenz weiters eine zweite Polynucleotidsequenz umfasst, die für das IND1-Polypeptid kodiert, worin die zweite Polynucleotidsequenz in der Antisense-Ausrichtung operabel an einen zweiten Promotor gebunden ist.

20. Verfahren nach Anspruch 18, worin die Polynucleotidsequenz weiters eine zweite Polynucleotidsequenz umfasst, die für das IND1-Polypeptid kodiert, worin die zweite Polynucleotidsequenz in der Antisense-Ausrichtung operabel an einen zweiten Promotor gebunden ist.

21. Pflanze nach Anspruch 17, bei Abhängigkeit von Anspruch 13, worin die Pflanze weiters ein zweites Polynucleotid mit einer Identität von zumindest 60 % mit zumindest 200 zusammenhängenden Nucleotiden einer Sequenz umfasst, die für Seq.-ID Nr. 2 kodiert, worin die zweite Polynucleotidsequenz in der Antisense-Ausrichtung operabel an einen zweiten Promotor gebunden ist.

22. Pflanze nach Anspruch 12 oder 13, worin die Lignifikation in Valve-Randzellen reduziert ist.

23. Verfahren nach Anspruch 14, worin die Lignifikation in Valve-Randzellen reduziert ist.

24. Pflanze nach Anspruch 12, worin der Promotor wie in Anspruch 10 oder Anspruch 11 definiert ist.

25. Verfahren nach Anspruch 14, worin der Promotor wie in Anspruch 10 oder Anspruch 11 definiert ist.

26. Pflanze nach Anspruch 13, worin der Promotor wie in Anspruch 8 oder 10 definiert ist.

27. Verfahren nach Anspruch 14, worin die rekombinante Expressionskassette unter Verwendung von Agrobacterium in die Pflanze eingeführt wird.

28. Verfahren nach Anspruch 14, worin das IND1-Polypeptid Seq.-ID Nr. 2 umfasst.

29. Verfahren nach Anspruch 14, worin das IND1-Polynucleotid Positionen von etwa 2765 bis etwa 3361 aus Seq.-ID Nr. 1 umfasst.

30. Verfahren nach Anspruch 14, worin das IND1-Polynucleotid Seq.-ID Nr. 1 umfasst.

31. Pflanze nach Anspruch 13, worin die Sequenz Seq.-ID Nr. 1 ist.

32. Pflanze nach Anspruch 13, worin das Polynucleotid eine Identität von zumindest 60 % mit einer Nucleotidsequenz aufweist, die für Seq.-ID Nr. 2 kodiert.

33. Pflanze nach Anspruch 13, worin die Pflanze im Vergleich zu einer Pflanze, welche die Expressionskassette nicht umfasst, durch verzögerte Samen-Dehiszenz **gekennzeichnet** ist.

34. Verfahren zur Verzögerung der Frucht-Dehiszenz bei einer Pflanze, wobei das Verfahren Folgendes umfasst:
(i) das Unterdrücken der Expression eines IND1-Gens oder eines Polypeptids mit einer Identität von zumindest 60 % mit Seq.-ID Nr. 2 in der Pflanze durch Einführen einer rekombinanten Expressionskassette, die eine Polynucleotidsequenz umfasst, die eine Identität von zumindest etwa 70 % mit Positionen von etwa 1 bis etwa 2764 oder von etwa 3362 bis etwa 3586 aus Seq.-ID Nr. 1 besitzt, in die Pflanze; sowie
(ii) das Auswählen einer Pflanze mit verzögerter Frucht-Dehiszenz im Vergleich zu einer Pflanze, bei der die Expression des IND1-Gens oder des Polypeptids nicht unterdrückt ist.

35. Verfahren nach Anspruch 34, Teil (i), worin die Polynucleotidsequenz Positionen von etwa 1 bis etwa 2764 oder von etwa 3362 bis etwa 3856 aus Seq.-ID Nr. 1 umfasst.

36. Verfahren nach Anspruch 34, worin die Lignifikation in Valve-Randzellen reduziert ist.

37. Verfahren zur Verzögerung der Frucht-Dehiszenz bei einer Pflanze, wobei das Verfahren folgende Schritte umfasst:
(i) Behandlung der Samen oder des Pflanzenmaterials mit einer mutagenen chemischen Substanz oder mit ionisierender Strahlung;
(ii) Identifikation von Pflanzen mit einem mutierten IND1-Gen, worin das IND1-Gen bevor es mutiert wird, für ein Polypeptid mit einer Sequenzidentität von zumindest 60 % mit Seq.-ID Nr. 2 kodiert; sowie
(iii) Auswählen einer Pflanze mit verzögerter Frucht-Dehiszenz im Vergleich zu einer Pflanze, bei der das IND1-Gen nicht mutiert ist.

38. Verfahren nach Anspruch 34, Teil (i), worin der Suppressionsschritt das Einführen einer rekombinanten Expressionskassette, wie in einem der Ansprüche 5 bis 7 definiert, in die Pflanze umfasst.

39. Verfahren nach Anspruch 38, worin das Polynucleotid in der Antisense-Ausrichtung operabel an den Promotor gebunden ist.

40. Verfahren nach Anspruch 38, worin das Polynucleotid in der Sense-Ausrichtung operabel an den Promotor gebunden ist.

41. Verfahren nach Anspruch 40, worin das Polynucleotid weiters ein zweites Polynucleotid umfasst, das eine Identität von zumindest 60 % mit zumindest 200 zusammenhängenden Nucleotiden einer Sequenz aufweist, die für Seq.-ID Nr. 2 kodiert, worin das zweite Polynucleotid in der Antisense-Ausrichtung operabel an einen zweiten Promotor gebunden ist.

42. Verfahren nach Anspruch 38, worin es sich bei dem Promotor um ein dehiszenzzonenselektives Regulationselement handelt.

43. Verfahren nach Anspruch 38, worin die rekombinante Expressionskassette unter Verwendung von Agrobacterium in die Pflanze eingeführt wird.

44. Pflanze, umfassend ein modifiziertes Polynucleotid mit einer Identität von zumindest 65 % mit Seq.-ID Nr. 1, worin die Pflanze im Vergleich zu einer Pflanze, der das modifizierte Polynucleotid fehlt, eine verzögerte Frucht-Dehiszenz aufweist.

45. Pflanze nach Anspruch 44, worin das modifizierte Polynucleotid eine Identität von zumindest 70 % mit Seq.-ID Nr. 1 aufweist.

46. Verfahren zur Erzeugung einer Pflanze mit verzögerter Frucht-Dehiszenz; wobei das Verfahren das Einführen einer rekombinanten Expressionskassette, die einen Promotor umfasst, der operabel an ein Polynucleotid gebunden ist, das eine Identität von zumindest 60 % mit zumindest 200 zusammenhängenden Nucleotiden aus Seq.-ID Nr. 1 umfasst, in die Pflanze sowie das Auswählen einer Pflanze mit verzögerter Frucht-Dehiszenz im Vergleich zu einer Pflanze, der die rekombinante Expressionskassette fehlt, umfasst.

47. Verfahren nach Anspruch 46, umfassend das Exprimieren eines Sense-Polynucleotids, das eine Identität von zumindest 65 % mit zumindest 200 zusammenhängenden Nucleotiden aus Seq.-ID Nr. 1 aufweist, in der Pflanze sowie das Exprimieren eines Antisense-Polynucleotids, das eine Identität von zumindest 65 % mit zumindest 200 zusammenhängenden Nucleotiden aus Seq.-ID Nr. 1 aufweist, in der Pflanze.

## Revendications

1. Cassette d'expression comprenant un promoteur opérativement enchaîné à une séquence de polynucléotide IND1, ou son complément, codant pour un polypeptide IND1 au moins 70% identique à SEQ ID NO:2.

2. Cassette d'expression de la revendication 1, où le polypeptide IND1 comprend SEQ ID NO:2.

3. Cassette d'expression de la revendication 1, où le polynucléotide IND1 comprend des positions d'environ 2765 à environ 3361 de SEQ ID NO:1.

4. Cassette d'expression de la revendication 3, où le polynucléotide IND1 comprend SEQ ID NO:1.

5. Cassette d'expression comprenant un promoteur hétérologue opérativement enchaîné à un polynucléotide ou son complément où le polynucléotide est identique à au moins 60% avec au moins 200 nucléotides contigus d'une séquence codant pour SEQ ID NO:2.

6. Cassette d'expression de la revendication 5, où la séquence est SEQ ID NO:1.

7. Cassette d'expression de la revendication 5, où le polynucléotide est identique à au moins 60% avec une séquence de nucléotides codant pour SEQ ID NO:2.

8. Cassette d'expression de l'une quelconque des revendications 1 à 7 où le promoteur est constitutif.

9. Cassette d'expression de l'une quelconque des revendications 1 à 7, où le promoteur est spécifique du tissu.

10. Cassette d'expression de la revendication 9, où le promoteur est un promoteur spécifique de la zone de déhiscence.

11. Cassette d'expression de la revendication 10, où le promoteur comprend des positions d'environ 1 à environ 2764 ou d'environ 3362 à environ 3856 de SEQ ID NO:1.

12. Plante comprenant une cassette d'expression recombinante comprenant un promoteur opérativement enchaîné à une séquence d'un polynucléotide IND1 codant pour un polypeptide IND1 identique à au moins 70% avec SEQ ID NO:2.

13. Plante comprenant une cassette d'expression recombinante telle que définie à la revendication 5.

14. Méthode pour retarder la déhiscence d'un fruit dans une plante, la méthode consistant à supprimer l'expression d'un acide nucléique de IND1 dans la plante par introduction dans la plante d'une cassette d'expression recombinante comprenant un promoteur opérativement enchaîné à une séquence de polnucléotide codant pour un polypeptide IND1 identique à au moins 70% avec SEQ ID NO:2.

15. Plante de la revendication 12 ou 13, où la séquence de polynucléotide est opérativement enchaînée au promoteur à l'orientation antisens.

16. Méthode de la revendication 14, où la séquence du polynucléotide est opérativement enchaînée au promoteur à l'orientation antisens.

17. Plante de la revendication 12 ou 13, où la séquence de polynucléotide est opérativement enchaînée au promoteur à l'orientation de sens.

18. Méthode de la revendication 14, où la séquence du polynucléotide est opérativement enchaînée au promoteur à l'orientation de sens.

19. Plante de la revendication 17 dépendante de la revendication 12, où la séquence du polynucléotide comprend de plus une seconde séquence de polynucléotide codant pour le polypeptide IND1 où la seconde séquence de polynucléotide est opérativement enchaînée à un second promoteur à l'orientation antisens.

20. Méthode de la revendication 18, où la séquence du polynucléotide comprend de plus une seconde séquence de polynucléotide codant pour le polypeptide IND1 où la seconde séquence de polynucléotide est opérativement enchaînée à un second promoteur à l'orientation antisens.

21. Plante de la revendication 17 dépendante de la revendication 13, où la plante comprend de plus un second polynucléotide identique à au moins 60% avec au moins 200 nucléotides contigus d'une séquence codant pour SEQ ID NO:2, où la seconde séquence de polynucléotide est opérativement enchaînée à un second promoteur à l'orientation antisens.

22. Plante de la revendication 12 ou 13, où la lignification est réduite dans les cellules du bord de la valve.

23. Méthode de la revendication 14, où la lignification est réduite dans les cellules du bord de la valve.

24. Plante de la revendication 12, où le promoteur est tel que défini à la revendication 10 ou à la revendication 11.

25. Méthode de la revendication 14, où le promoteur est tel que défini à la revendication 10 ou la revendication 11.

26. Plante de la revendication 13, où le promoteur est tel que défini à la revendication 8 ou 10.

27. Méthode de la revendication 14 où la cassette d'expression recombinante est introduite dans la plante en utilisant *Agrobacterium.*

28. Méthode de la revendication 14, où le polypeptide IND1 comprend SEQ ID NO:2.

29. Méthode de la revendication 14, où le polynucléotide IND1 comprend des positions d'environ 2765 à environ 3361 de SEQ ID NO:1.

30. Méthode de la revendication 14, où le polynucléotide IND1 comprend SEQ ID NO:1.

31. Plante de la revendication 13, où la séquence est SEQ ID NO:1.

32. Plante de la revendication 13, où le polynucléotide est identique à au moins 60% avec une séquence de nucléotides codant pour SEQ ID NO:2.

33. Plante de la revendication 13, où la plante est **caractérisée par** une déhiscence retardée de la graine en comparaison avec une plante qui ne comprend pas la cassette d'expression.

34. Méthode pour retarder la déhiscence d'un fruit dans une plante, la méthode consistant à:
(i) supprimer l'expression d'un gène IND1 ou d'un polypeptide identique à au moins 60% avec SEQ ID NO:2 dans la plante par introduction dans la plante d'une cassette d'expression recombinante comprenant une séquence de polynucléotide identique à au moins 70% avec les positions d'environ 1 à environ 2764 ou d'environ 3362 à environ 3586 de SEQ ID NO:1; et
(ii) sélectionner une plante avec une déhiscence retardée du fruit en comparaison avec une plante dans laquelle l'expression du gène IND1 ou du polypeptide n'est pas supprimée.

35. Méthode de la revendication 34, partie (i) où la séquence du polynucléotide comprend des positions d'environ 1 à environ 2764 ou d'environ 3362 à environ 3856 de SEQ ID NO:1.

36. Méthode de la revendication 34, où la lignification est réduite dans les cellules de marge de valve.

37. Méthode pour retarder la déhiscence d'un fruit dans une plante, la méthode comprenant les étapes de:
(i) traiter les graines ou le matériel de la plante avec une substance chimique mutagène ou avec un rayonnement ionisant;
(ii) identifier des plantes avec un gène *IND1* mutant où le gène *IND1,* avant d'être muté, code pour un polypeptide ayant au moins 60% d'identité de séquence avec SEQ ID NO:2; et
(iii) sélectionner une plante avec déhiscence retardée du fruit en comparaison avec une plante dans laquelle le gène *IND1* n'est pas muté.

38. Méthode de la revendication 34, partie (i) où l'étape de suppression consiste à introduire dans la plante une cassette d'expression recombinante telle que définie dans l'une quelconque des revendications 5 à 7.

39. Méthode de la revendication 38, où le polynucléotide est opérativement enchaîné au promoteur à l'orientation antisens.

40. Méthode de la revendication 38, où le polynucléotide est opérativement enchaîné au promoteur à l'orientation de sens.

41. Méthode de la revendication 40, où le polynucléotide comprend de plus un second polynucléotide identique à au moins 60% avec au moins 200 nucléotides contigus d'une séquence codant pour SEQ ID NO:2, où le second polynucléotide est opérativement enchaîné à un second promoteur à l'orientation antisens.

42. Méthode de la revendication 38, où le promoteur est un élément régulateur sélectif de la zone de déhiscence.

43. Méthode de la revendication 38, où la cassette d'expression recombinante est introduite dans la plante en utilisant Agrobacterium.

44. Plante comprenant un polynucléotide modifié identique au moins à 65% avec SEQ ID NO:1, où la plante présente une déhiscence retardée du fruit en comparaison avec une plante manquant du polynucléotide modifié.

45. Plante de la revendication 44, où le polynucléotide modifié est identique à 70% avec SEQ ID NO:1.

46. Méthode pour créer une plante avec déhiscence retardée du fruit, la méthode consistant à introduire dans ladite plante une cassette d'expression recombinante comprenant un promoteur opérativement enchaîné à un polynucléotide qui est identique à au moins 60% avec au moins 200 nucléotides contigus de SEQ IS NO:1 et à sélectionner une plante avec une déhiscence retardée du fruit en comparaison avec une plante manquant de la cassette d'expression recombinante.

47. Méthode de la revendication 46 comprenant l'expression dans la plante d'un polynucléotide de sens identique à au moins 65% avec au moins 200 nucléotides contigus de SEQ IS NO:1 et l'expression dans la plante d'un polynucléotide antisens identique à au moins 65% avec au moins 200 nucléotides contigus de SEQ ID NO:1.


### SEQUENCE LISTING

<110> Liljegren, Sarah
   Yanofsky, Martin F.
   The Regents of the University of California
<120> Control of Fruit Dehiscence in Arabidopsis by INDEHISCENT1 Genes
<130> 19452A-000700US
<140> WO PCT/US01/11967
   <141> 2001-04-13
<150> US 09/548,971
   <151> 2000-04-13
<160> 9
<170> PatentIn Ver. 2.1
<210> 1
   <211> 3856
   <212> DNA
   <213> Arabidopsis thaliana
<220>
   <223> INDEHISCENT1 (IND1) genomic
<220>
   <221> CDS
   <222> (2765) .. (3361)
   <223> IND1
<400> 1
   ctctagacca tctactatcc ggttgttgac ccttaaagct tttgaagact actagaataa 60
   tgcaaatacc atatgtccat atccatcctt ttcttttgtt tgaactgaac attctaattt 120
   tgtaaaagaa aaaaccttat gttaatatca ccgtaggcaa aaaaaatatc tcatcatatt 180
   aaatttttat tataagatta tacattctct cgttgtaaga gttactccaa ttgcaagtgt 240
   tgtattaact aataaaaagg acgaaagtag gaagcttata attaattgat gttgcatagt 300
   actggtatat tgttgatgaa tataacaagt atgaacatta atgcatgaaa cggggtattt 360
   tgtcttgaac tcattaaagg caatgtgaaa agaagatgtg aggtctcatt ttgaaaattt 420
   atcttctagc tttgtcgatt ttaaatctat gaaatgaacg caacatatag aaatttcatg 480
   tggacaacga catttagacg gtatcttaat tagaccgatt aattagtaat atacttatat 540
   atataattag tggtgattat aagtttactt atccacttga gaatttaaac aatgggcaat 600
   accttaatgt cgaaagaagc cgtccccact tcgtgtaatg agttatgggg gagagatcct 660
   gttaaatcgt caaataaaac aacttaagaa ctagaaattg acaccaaaaa tcataaagag 720
   aacgttgaag aagtcattta tcgtatccag ctcatatttc ctagctaaga tcaaatcaag 780
   gccgttgaaa gggcttgtaa gaaaatgtcg aagaaaccgt ggggtttaga agaaagacaa 840
   gaaatagaag aacaatgatg ttaaattgcc tattttggtg tataggagtt gtcaaaagag 900
   gagagagaga agaaaattag gtcaaaataa tgagcactaa aaatggagac atgtgttgag 960
   taactattac aagagcgact tatgcttcct tatggcaatg atatccaaac caaagtgcaa 1020
   cgctcctttt ttgccctaat ttcgtaaagt ctctctcctt cttcgtcctt aggaaaaacc 1080
   ctagaaattt aatcccttgt tcttgatctt gctttttgag taaccatgat tttgaccaca 1140
   cactatttct tctatctttt gtggtctata ggattttgct ttatatgtgt ttcttgtatt 1200
   gctccgtacg tacgtatacg aatttaaatg gttataacaa ggtttatata aactagcaca 1260
   aatgagtcca tgaaatttgt tagcgaaaaa ggtagaaata tattgagtct ttaaacggca 1320
   atatatataa ttttgctgca aaacttagct ttaatcatga tctaatgata ttttctttaa 1380
   tttcctttgc caaattaatc acatgcacgg atttttggca agttatgtgt cgaattcttc 1440
   cattcacaca acactaaact taattagaac tctaggaaat attttaaaat gacaacttta 1500
   tcgaaaaaaa tttagttatg aaaacaattc cagaattaaa catgagctat ataatttaag 1560
   ataaaatgaa gtaatattga tatgtatgta ataacatatc tgattgcggt aaaaaaaaac 1620
   atatctgatt aaattgttca tgcaggccca tgtcactatg atgtcatcac gtttttattt 1680
   tcacaataac taatatatat tcaaaaaaat agttttgtca gattaaattt tttttggtgg 1740
   tcagctttct ccaacctact aaactagttt ggaatgttct cttctttatt tttctttttc 1800
   ttgatttctt atgtttttta tttatggaat tttaagacgg attgtttagg tcgtttctct 1860
   cttttcttgt tttctaaagt tacttttgta aactcatctc ctcccaatta gacagtcaat 1920
   catatagtta tcttttaata tatgtctagt tgataaaaaa aatgaaaaaa tactggtggt 1980
   agttctacta atgtttgtgt aaaaaatctg atattatgaa tctaatcaat ttctttgatc 2040
   gtataatgtg ggttaaattt agtaattttt tacataaata agaactgtaa tgttgatgta 2100
   tattggggaa tcagtatatt agcttgggta actatacttc tggaaatact tgaagattta 2160
   actatttgca aaattataat ttagtcccga aaaatacaga cgacgggaca cgacaacata 2220
   taagcaggtt tgaatcttgg aaaattttgt atacataacc tatataaata ctaatgttct 2280
   ggttgggttc aaaagccttt tcaaaagttc cattttttaa attcaaggac attttacata 2340
   ggaaataagt tgagtcataa aaaataatgg ttattttgta aggttttttt tttgattaaa 2400
   acgcacatat taagaagtta gttttttttc actaccaaat atcaattaat ttaaaaccat 2460
   gcaaccattc ataaaacaat actattaaag aatataaata atcacaaaat attaaataca 2520
   cttaaaattt acatataaat ttacaaaaca tctaattaat tgaaacagaa aggaaaaggt 2580
   aaaatatatc ataaaatgag acatatatcc tataaaaaaa aaatgaggca tatgaagtaa 2640
   ataataagag acatgcatgt aagcattcgg ttaattaatc gagtcaaaga tatatatcag 2700
   taaatacata tgtgtatatt tctggaaaaa gaatatatat attgagaaat aagaaaagat 2760
   gaaa atg gaa aat ggt atg tat aaa aag aaa gga gtg tgc gac tct tgt  2809
        Met Glu Asn Gly Met Tyr Lys Lys Lys Gly Val Cys Asp Ser Cys
        1               5                   10                  15
   gtc tcg tcc aaa agc aga tcc aac cac agc ccc aaa aga agc atg atg   2857
   Val Ser Ser Lys Ser Arg Ser Asn His Ser Pro Lys Arg Ser Met Met
                   20                  25                  30
   gag cct cag cct cac cat ctc ctc atg gat tgg aac aaa gct aat gat   2905
   Glu Pro Gln Pro His His Leu Leu Met Asp Trp Asn Lys Ala Asn Asp
               35                  40                  45
   ctt ctc aca caa gaa cac gca gct ttt ctc aat gat cct cac cat ctc   2953
   Leu Leu Thr Gln Glu His Ala Ala Phe Leu Asn Asp Pro His His Leu
           50                  55                  60
   atg tta gat cca cct ccc gaa acc cta att cac ttg gac gaa gac gaa   3001
   Met Leu Asp Pro Pro Pro Glu Thr Leu Ile His Leu Asp Glu Asp Glu
       65                  70                  75
   gag tac gat gaa gac atg gat gcg atg aag gag atg cag tac atg atc   3049
   Glu Tyr Asp Glu Asp Met Asp Ala Met Lys Glu Met Gln Tyr Met Ile
   80                  85                  90                  95
   gcc gtc atg cag ccc gta gac atc gac cct gcc acg gtc cct aag ccg   3097
   Ala Val Met Gln Pro Val Asp Ile Asp Pro Ala Thr Val Pro Lys Pro
                   100                 105                 110
   aac cgc cgt aac gta agg ata agc gac gat cct cag acg gtg gtt gct   3145
   Asn Arg Arg Asn Val Arg Ile Ser Asp Asp Pro Gln Thr Val Val Ala
               115                 120                 125
   cgt cgg cgt cgg gaa agg atc agc gag aag atc cga att ctc aag agg   3193
   Arg Arg Arg Arg Glu Arg Ile Ser Glu Lys Ile Arg Ile Leu Lys Arg
           130                 135                 140
   atc gtg cct ggt ggt gcg aag atg gac aca gct tcc atg ctc gac gaa   3241
   Ile Val Pro Gly Gly Ala Lys Met Asp Thr Ala Ser Met Leu Asp Glu
       145                 150                 155
   gcc ata cgt tac acc aag ttc ttg aaa cgg cag gtg agg att ctt cag   3289
   Ala Ile Arg Tyr Thr Lys Phe Leu Lys Arg Gln Val Arg Ile Leu Gln
   160                 165                 170                 175
   cct cac tct cag att gga gct cct atg gct aac ccc tct tac ctt tgt   3337
   Pro His Ser Gln Ile Gly Ala Pro Met Ala Asn Pro Ser Tyr Leu Cys
                   180                 185                 190
   tat tac cac aac tcc caa ccc tga tgaactacac agaagctcgc tagctagaca  3391
   Tyr Tyr His Asn Ser Gln Pro
               195
   tttggtgtca tcctctcaac ctttttcatg ttgatatatt atatatagat gcataaagat 3451
   tcgatccaag attgtatggg tgttttaata ttattattct aagatatatg atgtacaatt 3511
   gtgtaccaag tttctttatc ttgatatcat atgcataaat aattggtgaa taaaaagaag 3571
   atattgattg taaacaaaaa aaagaagata ttgattgtta attagggttt gatcattctg 3631
   tatgaaagct ttggcctgca aattaatttt cgatatatat atatatatat ggagaatata 3691
   tatcaaatac ttttttaatt tgactataat ttgtatcaat tatctgaatc tgatgagtgt 3751
   aggttatata tggattagca aaaaagaaaa caaccattat tacgcaccta cattaaaaat 3811
   catccaccaa agaagaaacc atcctcaaga gggttccctc tagag                 3856
<210> 2
   <211> 198
   <212> PRT
   <213> Arabidopsis thaliana
   <223> INDEHISCENT1 (IND1) protein
<400> 2
   Met Glu Asn Gly Met Tyr Lys Lys Lys Gly Val Cys Asp Ser Cys Val
    1               5                  10                  15
   Ser Ser Lys Ser Arg Ser Asn His Ser Pro Lys Arg Ser Met Met Glu
               20                  25                  30
   Pro Gln Pro His His Leu Leu Met Asp Trp Asn Lys Ala Asn Asp Leu
           35                  40                  45
   Leu Thr Gln Glu His Ala Ala Phe Leu Asn Asp Pro His His Leu Met
       50                  55                  60
   Leu Asp Pro Pro Pro Glu Thr Leu Ile His Leu Asp Glu Asp Glu Glu
   65                  70                  75                  80
   Tyr Asp Glu Asp Met Asp Ala Met Lys Glu Met Gln Tyr Met Ile Ala
                   85                  90                  95
   Val Met Gln Pro Val Asp Ile Asp Pro Ala Thr Val Pro Lys Pro Asn
               100                 105                 110
   Arg Arg Asn Val Arg Ile Ser Asp Asp Pro Gln Thr Val Val Ala Arg
           115                 120                 125
   Arg Arg Arg Glu Arg Ile Ser Glu Lys Ile Arg Ile Leu Lys Arg Ile
       130                 135                 140
   Val Pro Gly Gly Ala Lys Met Asp Thr Ala Ser Met Leu Asp Glu Ala
   145                 150                 155                 160
   Ile Arg Tyr Thr Lys Phe Leu Lys Arg Gln Val Arg Ile Leu Gln Pro
                   165                 170                 175
   His Ser Gln Ile Gly Ala Pro Met Ala Asn Pro Ser Tyr Leu Cys Tyr
               180                 185                 190
   Tyr His Asn Ser Gln Pro
           195
<210> 3
   <211> 2765
   <212> DNA
   <213> Arabidopsis thaliana
<220>
   <223> 5' untranslated region IND1 promoter
<400> 3
   ctctagacca tctactatcc ggttgttgac ccttaaagct tttgaagact actagaataa 60
   tgcaaatacc atatgtccat atccatcctt ttcttttgtt tgaactgaac attctaattt 120
   tgtaaaagaa aaaaccttat gttaatatca ccgtaggcaa aaaaaatatc tcatcatatt 180
   aaatttttat tataagatta tacattctct cgttgtaaga gttactccaa ttgcaagtgt 240
   tgtattaact aataaaaagg acgaaagtag gaagcttata attaattgat gttgcatagt 300
   actggtatat tgttgatgaa tataacaagt atgaacatta atgcatgaaa cggggtattt 360
   tgtcttgaac tcattaaagg caatgtgaaa agaagatgtg aggtctcatt ttgaaaattt 420
   atcttctagc tttgtcgatt ttaaatctat gaaatgaacg caacatatag aaatttcatg 480
   tggacaacga catttagacg gtatcttaat tagaccgatt aattagtaat atacttatat 540
   atataattag tggtgattat aagtttactt atccacttga gaatttaaac aatgggcaat 600
   accttaatgt cgaaagaagc cgtccccact tcgtgtaatg agttatgggg gagagatcct 660
   gttaaatcgt caaataaaac aacttaagaa ctagaaattg acaccaaaaa tcataaagag 720
   aacgttgaag aagtcattta tcgtatccag ctcatatttc ctagctaaga tcaaatcaag 780
   gccgttgaaa gggcttgtaa gaaaatgtcg aagaaaccgt ggggtttaga agaaagacaa 840
   gaaatagaag aacaatgatg ttaaattgcc tattttggtg tataggagtt gtcaaaagag 900
   gagagagaga agaaaattag gtcaaaataa tgagcactaa aaatggagac atgtgttgag 960
   taactattac aagagcgact tatgcttcct tatggcaatg atatccaaac caaagtgcaa 1020
   cgctcctttt ttgccctaat ttcgtaaagt ctctctcctt cttcgtcctt aggaaaaacc 1080
   ctagaaattt aatcccttgt tcttgatctt gctttttgag taaccatgat tttgaccaca 1140
   cactatttct tctatctttt gtggtctata ggattttgct ttatatgtgt ttcttgtatt 1200
   gctccgtacg tacgtatacg aatttaaatg gttataacaa ggtttatata aactagcaca 1260
   aatgagtcca tgaaatttgt tagcgaaaaa ggtagaaata tattgagtct ttaaacggca 1320
   atatatataa ttttgctgca aaacttagct ttaatcatga tctaatgata ttttctttaa 1380
   tttcctttgc caaattaatc acatgcacgg atttttggca agttatgtgt cgaattcttc 1440
   cattcacaca acactaaact taattagaac tctaggaaat attttaaaat gacaacttta 1500
   tcgaaaaaaa tttagttatg aaaacaattc cagaattaaa catgagctat ataatttaag 1560
   ataaaatgaa gtaatattga tatgtatgta ataacatatc tgattgcggt aaaaaaaaac 1620
   atatctgatt aaattgttca tgcaggccca tgtcactatg atgtcatcac gtttttattt 1680
   tcacaataac taatatatat tcaaaaaaat agttttgtca gattaaattt tttttggtgg 1740
   tcagctttct ccaacctact aaactagttt ggaatgttct cttctttatt tttctttttc 1800
   ttgatttctt atgtttttta tttatggaat tttaagacgg attgtttagg tcgtttctct 1860
   cttttcttgt tttctaaagt tacttttgta aactcatctc ctcccaatta gacagtcaat 1920
   catatagtta tcttttaata tatgtctagt tgataaaaaa aatgaaaaaa tactggtggt 1980
   agttctacta atgtttgtgt aaaaaatctg atattatgaa tctaatcaat ttctttgatc 2040
   gtataatgtg ggttaaattt agtaattttt tacataaata agaactgtaa tgttgatgta 2100
   tattggggaa tcagtatatt agcttgggta actatacttc tggaaatact tgaagattta 2160
   actatttgca aaattataat ttagtcccga aaaatacaga cgacgggaca cgacaacata 2220
   taagcaggtt tgaatcttgg aaaattttgt atacataacc tatataaata ctaatgttct 2280
   ggttgggttc aaaagccttt tcaaaagttc cattttttaa attcaaggac attttacata 2340
   ggaaataagt tgagtcataa aaaataatgg ttattttgta aggttttttt tttgattaaa 2400
   acgcacatat taagaagtta gttttttttc actaccaaat atcaattaat ttaaaaccat 2460
   gcaaccattc ataaaacaat actattaaag aatataaata atcacaaaat attaaataca 2520
   cttaaaattt acatataaat ttacaaaaca tctaattaat tgaaacagaa aggaaaaggt 2580
   aaaatatatc ataaaatgag acatatatcc tataaaaaaa aaatgaggca tatgaagtaa 2640
   ataataagag acatgcatgt aagcattcgg ttaattaatc gagtcaaaga tatatatcag 2700
   taaatacata tgtgtatatt tctggaaaaa gaatatatat attgagaaat aagaaaagat 2760
   gaaaa                                                             2765
<210> 4
   <211> 496
   <212> DNA
   <213> Arabidopsis thaliana
<220>
   <223> 3' untranslated region IND1 promoter
<400> 4
   atgaactaca cagaagctcg ctagctagac atttggtgtc atcctctcaa cctttttcat 60
   gttgatatat tatatataga tgcataaaga ttcgatccaa gattgtatgg gtgttttaat 120
   attattattc taagatatat gatgtacaat tgtgtaccaa gtttctttat cttgatatca 180
   tatgcataaa taattggtga ataaaaagaa gatattgatt gtaaacaaaa aaaagaagat 240
   attgattgtt aattagggtt tgatcattct gtatgaaagc tttggcctgc aaattaattt 300
   tcgatatata tatatatata tggagaatat atatcaaata cttttttaat ttgactataa 360
   tttgtatcaa ttatctgaat ctgatgagtg taggttatat atggattagc aaaaaagaaa 420
   acaaccatta ttacgcacct acattaaaaa tcatccacca aagaagaaac catcctcaag 480
   agggttccct ctagag                                                 496
<210> 5
   <211> 5622
   <212> DNA
   <213> Arabidopsis thaliana
<220>
   <223> SHATTERPROOF1 (SHP1) genomic
<220>
   <221> modified_base
   <222> (935)..(941)
   <223> n = any nucleotide
<400> 5
   agatctgcaa cagtgaaaag agaaaacaaa atggacttga agaggttttg acaatgccag 60
   agataatgct tattccctaa tatgttgcca gccaagtgtc aaattggctt tttaaatatg 120
   gatttctgta tcagtggtca tatttgtgga tccaacgtat tcatcatcaa gttctcaagt 180
   ttgctttcag tgcaattcta attcacacgt ttaactttaa catgcatgtc attataatta 240
   cttcttcact aagacacaat acggcaaacc tttcagatta tattaatctc cataaatgaa 300
   ataattaacc tcataatcaa gattcaatgt ttctaaatat atatggacaa aatttacacg 360
   gaagattaga tacgtatatt agtagattta gtctttcgtt tgtgcgataa gattaaccac 420
   ctcatagata gtaatatcat tgtcaaattc ctctcggttt agtcgctaaa ttgtatcttt 480
   tttaagccta aaagtagtgt attcgcatat gacttatcgt cctaactttt tttttaatta 540
   acaaaaaaat cgaaaagaaa ataatctgtt aaatattttt taagtactcc attaagttta 600
   gtttctattt aaaaaatgct tgaaatttga cagttatgtt caacaatttt gaatcatgag 660
   cgatgtctag atactcagaa tttaatcaag atgtcttatc aaatttgttg tcactcgagg 720
   acccacgcaa aagaaaagac taatatgatt tttatttggt ctggatattt ttgtagagga 780
   tgaaactaag agagtgaaag attcgaaatc cacaatgttc aagagagctc aaagcaaaaa 840
   gaaaaatgaa gatgaaggac taaagaacaa taagcaacta cttataccct atttccataa 900
   aggattcagg tactaggaga agttgaggca agttnnnnnn nattgattca aattttcatt 960
   tatttttaca atttaattca cctaagttat tatgcatttc tcatcattgg tacattttct 1020
   gtatagcgta tttacatata tgaaataaat taaatatgtc ctcacgttgc aagtagttaa 1080
   tgaatgtccc cacgcaaaaa aaaatccctc caaatatgtc caccttttct tttcttttta 1140
   attccaaaat taccataaac ttttggttta caaaagattt ctagaaattg aggaagatat 1200
   cctaaatgat tcatgaatcc ttcaataatc tgaagtttgc gatattttcg attttcttca 1260
   agagttgcga tatttgtaat ttggtgacct taaacttttt ttgataaaga gtaaacgttt 1320
   tttcttaaaa gtaaaacttg attttatgtt ttagggttct agctcaactt tgtattatat 1380
   ttcttgcaaa aagagttcgt taactgcatt cttcaacact ataaagtgat tatcaaaaac 1440
   atcttcatga acattaagaa aaacaatatt tggtttcggt tagagcttgg ttttgcttgg 1500
   cttgattcac atacccattc tagactttgg cataaatttg atacgataga gagtatctaa 1560
   tggtaatgca gaagggtaaa aaaaggaaga gagaaaaggt gagaaagatt accaaaaata 1620
   aggagtttca aaagatggtt ctgatgagaa acagagccca tccctctcct tttccccttc 1680
   ccatgaaaga aatcggatgg tcctccttca atgtcctcca cctactcttc tcttctttct 1740
   ttttttcttt cttattatta accatttaat taatttcccc ttcaatttca gtttctagtt 1800
   ctgtaaaaag aaaatacaca tctcacttat agatatccat atctatttat atgcatgtat 1860
   agagaataaa aaagtgtgag tttctaggta tgttgagtat gtgctgtttg gacaattgtt 1920
   agatgatctg tccatttttt tcttttttct tctgtgtata aatatatttg agcacaaaga 1980
   aaaactaata accttctgtt ttcagcaact agggtcttat aaccttcaaa gaaatattcc 2040
   ttcaattgaa aacccataaa ccaaaataga tattacaaaa ggaaagagag atattttcaa 2100
   gaacaacata attagaaaag cagaagcagc agttaagtgg tactgagata aatgatatag 2160
   tttctcttca agaacagttt ctcattaccc accttctcct ttttgctgat ctatcgtaat 2220
   cttgagaact caggtaaggt tgtgaatatt atgcaccatt cattaaccct aaaaataaga 2280
   gatttaaaat aaatgtttct tctttctctg attcttgtgt aaccaattca tgggtttgat 2340
   atgtttcttg gttattgctt atcaacaaag agatttgatc attataaagt agattaataa 2400
   ctcttaaaca cacaaagttt ctttattttt tagttacatc cctaattcta gaccagaaca 2460
   tggatttgat ctatttcttg gttatgtatc ttgatcagga aaagggattt gatcatcaag 2520
   attagccttc tctctctctc tctagatatc tttcttgaat ttagaaatct ttatttaatt 2580
   atttggtgat gtcatatatg gatcaatgga ggaaggtggg agtagtcacg acgcagagag 2640
   tagcaagaaa ctagggagag ggaaaataga gataaagagg atagagaaca caacaaatcg 2700
   tcaagttact ttctgcaaac gacgcaatgg tcttctcaag aaagcttatg aactctctgt 2760
   cttgtgtgat gccgaagttg ccctcgtcat cttctccact cgtggccgtc tctatgagta 2820
   cgccaacaac aggtacgctt ctcctactct atttcttgat cttgttttct taattttaac 2880
   taaacaagat cctagttcaa atgataacaa agtggggatt gagagccaag attagggttt 2940
   ggttaattta gaaaaccaga tttcacttgt tgatacattt aatatctctc tagctagatt 3000
   tagtactctc tcctctatat atgtgtgggt gtgtgtgtaa gtgtgtatat gtatgcaaat 3060
   gcaagaagaa gaagaaaaag ttatcttgtc ttctcaaatt ctgatcagct ttgaccttag 3120
   tttcactctt ttttctgcaa atcatttgaa cctgatgcat gtcagtttct acaatacact 3180
   tttaattttg acggcccatc aaatttccta gggtttactt cagtgaacaa aattgggttc 3240
   ttgacacgat ttagcatgta tatataaaaa taggggatga tcaagactta tgtaacctct 3300
   gtctggtgaa actagggaca aagtctactg atgagttgtc actagggatc catttgatca 3360
   tttaatccca acaaaaatga aacaaaattt tgagaattta tatgctgaag tttttcaacc 3420
   ctctttttta aataacttta tattatgtag atttgtattt agggtaattt gtccaactag 3480
   aagtcctaaa aatcaataaa cacacggatg actttgtcta acattgtatc agtcatcaaa 3540
   tgtaaaattg tacaaataat gaaattaaag atttagtctc ttttattttt tttgtttagg 3600
   gtgtatatat atatatatat gtatatttgt tgcattgata tatcaatgag agggagagaa 3660
   ctcagagaag tgtcggaaat taaaatggta cgagccaatt ggaatctctg gcattctgag 3720
   cttcatttgt ttgttattag aaaaaaaaaa aaaaaatcct ttaaagatac cttcatgatg 3780
   acattgaatc atgtaatata cacgatacat ggtctaattc ctcctcaaac cctaattacc 3840
   aatttcgaaa ccataatatt tactagtatg tttatatatc cttactttaa gacattgttt 3900
   gtttataata ccttgtgaat taagaaaaaa aaaaaaaaac ttgtggatct attcaagcca 3960
   tgtgttagaa taaatttata aattttctcc tcgtactggt cagatattgg tccaaactcc 4020
   aaagccttcc cttttcagga aaaaaaacat ttcgaaatta actctaatta atcaagaatt 4080
   tcctacaatg tatacatcta atgttttttc cgcgatctta cttattagtg tgaggggtac 4140
   aattgaaagg tacaagaaag cttgttccga tgccgtcaac cctccttccg tcaccgaagc 4200
   taatactcag gtaccaattt atattgtttg attctctttg ttttatcttc ttcttttcat 4260
   tatatatatg atcaacaaaa aatataacct acaaaaagag agagttcaag gaaatgcatt 4320
   gaaacggttt cgttatggtg tttgaataca tggatttttg aagtactatc agcaagaagc 4380
   ctctaagctt cggaggcaga ttcgagatat tcagaattca aataggtaat tcattaactt 4440
   ttcatgaact cttcgatttg gtattaggtc acttaatttg gtgtcggtcc aaaagtccgc 4500
   ttgtagtttt ctttagaagt tgttttgttt aatgttcatg tttacaaatt gaaggcatat 4560
   tgttggggaa tcacttggtt ccttgaactt caaggaactc aaaaacctag aaggacgtct 4620
   tgaaaaagga atcagccgtg tccgctccaa aaaggtaaaa tctacgttgc tctctctctg 4680
   tgtctctgtc tctctctcta tatatagtcc cttagtttat atagttcatc acccttttgt 4740
   gagaattttg cagaatgagc tgttagtggc agagatagag tatatgcaga agagggtaag 4800
   aacgtttctc ccattccaag taattagatc tttcttcgtc tttgtgaggg tttgagtttt 4860
   cccataaatc atgtgtagga aatggagttg caacacaata acatgtacct gcgagcaaag 4920
   gttagccacg ttctgttcca aatcttaatc tcaatatcta ctcttttctt cattgtataa 4980
   ctaagataac gtgaataaca agaaaacttt tgtttttggg tttaatagat agccgaaggc 5040
   gccagattga atccggacca gcaggaatcg agtgtgatac aagggacgac agtttacgaa 5100
   tccggtgtat cttctcatga ccagtcgcag cattataatc ggaactatat tccggtgaac 5160
   cttcttgaac cgaatcagca attctccggc caagaccaac ctcctcttca acttgtgtaa 5220
   ctcaaaacat gataacttgt ttcttcccct cataacgatt aagagagaga cgagagagtt 5280
   cattttatat ttataacgcg actgtgtatt catagtttag gttctaataa tgataataac 5340
   aaaactgttg tttctttgct taattacatc aacatttaaa tccaaagttc taaaacacgt 5400
   cgagatccaa agtttgtcat acaagattag acgcatacac gatcagttaa tagattttaa 5460
   gtgcctttta atatttacat atagttgcag cttcgattag atcatgtcca ccaaacactc 5520
   acaattagag acaagcaaaa ctataaacat tgatcataaa atgattacaa catgtccata 5580
   aattaattat ggattacaaa aataaaaact tacaaaagat ct                   5622
<210> 6
   <211> 6138
   <212> DNA
   <213> Arabidopsis thaliana
<220>
   <223> SHATTERPROOF2 (SHP2) genomic
<400> 6
   gaattcgtaa cagaatttag tgaataatat tgtaattacc aggcaaggac tctccaaacg 60
   gatagctcga atatcgttat taaagagtaa atgatccaat atgtaagcca ttgttgatca 120
   tctaacattg ttggactctc tattgctcga aatgatgcat acctaatcat ttattcagtt 180
   aactatcaag ttgcatttgt aaaaaccaaa catttaaatt cagatttgat atcacttaca 240
   gaggatagag aagcatgact ccaggcctgc atgcaacaag aaaaaggaag aaaataatgt 300
   taaaaatttg acaaatatag tgtttatttt tattatatga gacagaattt gaataaaatc 360
   ctacccaact agagcatcaa aacgttttgc aatcgcaata atgaaaccca ttttcttttt 420
   gagtttttac tcttctttca acagaaactt tctcaaacgt ctttagcact gtgacgttag 480
   atatatacac aaaagcttga aatttcttca agcaaaagaa tctttgtggg agttaaggca 540
   acaagccagg taaagaatct ccaacgcatt gttacgtttt catgaaccta tttattatat 600
   gttctaagaa agaaaaaaat atctcaaagt aaacgttgga aattttctga tgaagggaaa 660
   tccaaagtct tgggtttagt atccctatga atggtatttg gaatatgttt tcgtcaaaac 720
   aaaagattct tttctttttc acaagagtta gtgatcaata acttatgcac taattaatga 780
   gattggacgt atacacaatt tgattatgat acttgagtaa aaatcacctg tcctttaatt 840
   tggaaatctc tctttcttac ccatttatat actacttctt ttcattaaaa ttaaatttca 900
   attatcaatc atcgttcaat ttgataaaga tttaacattt tttgtcacag ggctagtaaa 960
   agcaatcttt acataattca tctttcttac atatatatat tacctttttc ttcattagta 1020
   ttctatttga ttatgattat tttgtcataa agctagtaaa ttaaacactc gatatgagaa 1080
   ttatattact tcacgctaat taactcttaa cacaacaaga actagtgcat attcaacttt 1140
   caaagcatat actatatatt gagaatatag accacgaaag tcaatcaaaa gacctaccag 1200
   ctctcatcaa gttctttctt gaaatgattt tgcagaattt ccaacttaat taattcgaca 1260
   tgaatgtgaa aatgtgtgtt gctcgttaag aaaattgaat agaagtacaa tgaaaatgat 1320
   gaggaatggg caaaacacaa aagagtttcc tttcgtaact acaattaatt aatgcaaatc 1380
   tgagaaaggg ttcatggata atgactacac acatgattag tcattccccg tgggctctct 1440
   gctttcattt actttattag tttcatcttc tctaattata ttgtcgcata tatgatgcag 1500
   ttcttttgtc taaattacgt aatatgatgt aattaattat caaaataaat attcaaattg 1560
   ccgttggact aacctaatgt ccaagattaa gacttgaaca taagaatttt ggaaaaacta 1620
   aaccagttat aatatatact cttaaattgc catttctgaa cacaaccaaa taataatata 1680
   tactatttac agtttttttt aattggcaag aacactgaaa tcttattcat tgtctcgctt 1740
   ggtagttgac aagttataac actcatattc atataacccc attctaacgt tgacgacgaa 1800
   cactcatata aaccacccaa attcttagca tattagctaa atattggttt aattggaaat 1860
   atttttttta tatataaaat gccaggtaaa tattaacgac atgcaatgta tataggagta 1920
   gggcaataaa aagaaaagga gaataaaaag ggattaccaa aaaaggaaag tttccaaaag 1980
   gtgattctga tgagaaacag agcccatacc tctctttttt cctctaaaca tgaaagaaaa 2040
   attggatggt cctccttcaa tgctctctcc ccacccaatc caaacccaac tgtcttcttt 2100
   ctttcttttt tcttctttct aatttgatat tttctaccac ttaattccaa tcaatttcaa 2160
   atttcaatct aaatgtatgc atatagaatt taattaaaag aattaggtgt gtgatatttg 2220
   agaaaatgtt agaagtaatg gtccatgttc tttctttctt tttccttcta taacacttca 2280
   gtttgaaaaa aaactaccaa accttctgtt ttctgcaaat gggtttttaa atacttccaa 2340
   agaaatattc ctctaaaaga aattataaac caaaacagaa accaaaaaca aaaaataaag 2400
   ttgaagcagc agttaagtgg tactgagata ataagaatag tatctttagg ccaatgaaca 2460
   aattaactct ctcataattc atcttcccat cctcacttct ctttctttct gatataatta 2520
   atcttgctaa gccaggtatg gttattgatg atttacactt ttttttaaaa gtttcttcct 2580
   tttctccaat caaattcttc agttaatcct tataaaccat ttctttaatc caaggtgttt 2640
   gagtgcaaaa ggatttgatc tatttctctt gtgtttatac ttcagctagg gcttatagaa 2700
   atggagggtg gtgcgagtaa tgaagtagca gagagcagca agaagatagg gagagggaag 2760
   atagagataa agaggataga gaacactacg aatcgtcaag tcactttctg caaacgacgc 2820
   aatggtttac tcaagaaagc ttatgagctc tctgtcttgt gtgacgctga ggttgctctt 2880
   gtcatcttct ccactcgagg ccgtctctac gagtacgcca acaacaggta cacatctttt 2940
   agctagatct tgattttgtt gaattttttt tctagaataa agtttcgact cttctggtgg 3000
   gtttttcaat ctttatggtc tctttatagt ttttttcctt agtttctctg aagctcaaat 3060
   ctctttaaaa atccccaaaa ttagggtttg tttaaaacta gggaacccta ctttaacttc 3120
   tttctcttag taaaaaagca gtgagggtct tctctgatca ttaattagca tcccccatac 3180
   cttgttccag tcactttttc tccacaaatc cttataacag tatctatata tgtatctatt 3240
   tatgtcagtt tgtacaagac acttcgatca atttgatgac ccatcaagtt ttatttctgc 3300
   agattgatca ttaggtttcc atcatagtaa tgaaaaagta gggttcttga taaaattata 3360
   ataatatata ttatttggct atataaaaaa gctatgtaga ttccttaaaa attgattcac 3420
   tagggagaga ctagtaggtg tttgtcttct gacacttctc taatcttttg gtgaatcctt 3480
   ttgttaaatc aagaaaatga atcagggaca aagcttattg ttgagtcact taattaatca 3540
   tccgatccat caatcaagaa aaataacgaa acagaaaatt ttgatttttg attgttattt 3600
   tctccacttc aagttgggga cttgtcattt ccgtttttct atacgtttcc agctattaac 3660
   agctcatgtt catttcacca ttttgattat ttgtctgctt tttaaagata aatgttttca 3720
   aaaatattgt ttttatttgc ttggctagtt aatactataa ttgaggttga tgtatgacta 3780
   taatctataa gtcaagtctc atatcatgga tctaagttaa aactagtaaa tttgtagttt 3840
   caatgtgaac tttcacaacg actaaagaac tgatctgaag tttataatgg acatgactaa 3900
   tttgattaac aaaagaggaa tgcattatgt atgtagaaac atgtgatata tatatgtttc 3960
   tattatcaaa agtgtagtta actttcttat ttcaaacacc ctcatgcttt agtagtatct 4020
   tacttttgac atttctcaac ttcagctttc cattatacaa cagcacaatg taaattactt 4080
   gtatatgaat atgaaagcat aacgttatgc aaagatttct agcttttctt tttctgtttt 4140
   gcaaaagatt tacaaatatc atgttcttgg taaaaacata cttgcctcag ccacatatgc 4200
   atgtaaatgt aatgttcaaa tattaattca ggaaaaacaa agaagaagca aaattagctt 4260
   ctagagtagg gaatctattg acttgacctg aaaatcactt ctttttctta aagcctagta 4320
   gtgaattttt taatctaatt aggccaaaat atatactagc ctaaaatata atttggattt 4380
   tgtgtcgtac ataaattggg accaattcca attaactaag agcatatgca attcaaattc 4440
   tttttatttt cttctccgat ttgctacttc tttcttttgt atgttttcaa attaggatta 4500
   cacttttttg gggaagtaca cattagggtc ttctcgaact ttgattatac atatatatat 4560
   atatatatat atataacttt gtgagatgtc actgttaata gataataggc aataacaata 4620
   atatccaaaa aagaaggcgc aaacaaatca tatactatat ggtactggtc cattcactat 4680
   tttgtcggtt gaatttaagg tttggcgtac aaactttgtt tcaaaccttt attattccgt 4740
   ctttctgtgt gttttgtata tccagaagat aaaaatatca atttctttaa cgacttcata 4800
   tatatatata tatatatata tatatatatt tttctcttct ggttttagtg tttgaatcca 4860
   acagttatag tttcgtgtgt ctttgtttta cttgtggtgg tttaagtttg agattttcac 4920
   cgattgcatc tatttacata tatagctacc acaaaaaaga ttgcatttta aaatcttttc 4980
   ctttgtgtga atgttgatga agtgtgagag gaacaataga aaggtacaag aaagcttgct 5040
   ccgacgccgt taaccctccg accatcaccg aagctaatac tcaggttagc ttttaattaa 5100
   tacacctagc tagctagttc gttaattact taatttcttc ttcttttagt tatctgacct 5160
   ttttttcacc tcttgtaaca atgatgggat cgaaattgat gaagtactat cagcaagagg 5220
   cgtctaaact ccggagacag attcgggaca ttcagaattt gaacagacac attcttggtg 5280
   aatctcttgg ttccttgaac tttaaggaac tcaagaacct tgaaagtagg cttgagaaag 5340
   gaatcagtcg tgtccgatcc aagaaggtac atcactaact ctccatcaat ctccttatca 5400
   ttgaatatat atccatctga ttcttgcccg ttatatttgg tttttctctc cagcacgaga 5460
   tgttagttgc agagattgaa tacatgcaaa aaagggtaaa agtaaaacct atcttccttc 5520
   acaatgaact acccctactt tattagcaac ttctctttct gatgatcatc ttttttattt 5580
   tctgttgtcg cttgcattgt aggaaatcga gctgcaaaac gataacatgt atctccgctc 5640
   caaggtttta tacataactc tttttggcat ttttgatcat catttttttc cggtagacaa 5700
   tctcttgatg tgcaaattct aaatatctct gcagattact gaaagaacag gtctacagca 5760
   acaagaatcg agtgtgatac atcaagggac agtttacgag tcgggtgtta cttcttctca 5820
   ccagtcgggg cagtataacc ggaattatat tgcggttaac cttcttgaac cgaatcagaa 5880
   ttcctccaac caagaccaac cacctctgca acttgtttga ttcagtctaa cataagcttc 5940
   tttcctcagc ctgagatcga tctatagtgt cacctaaatg cggccgcgtc cctcaacatc 6000
   tagtcgcaag ctgaggggaa ccactagtgt catacgaacc tccaagagac ggttacacaa 6060
   acgggtacat tgttgatgtc atgtatgaca atcgcccaag taagtatcca gctgtgttca 6120
   gaacgtacgt ccgaattc                                                6138
<210> 7
   <211> 51
   <212> PRT
   <213> Artificial Sequence
<220>
   <223> Description of Artificial Sequence:IND1 basic helix-loop-helix (bHLH) DNA binding domain
<400> 7
   Ile Ser Asp Asp Pro Gln Thr Val Val Ala Arg Arg Arg Arg Glu Arg
     1               5                  10                  15
   Ile Ser Glu Lys Ile Arg Ile Leu Lys Arg Ile Val Pro Gly Gly Ala
               20                  25                  30
   Lys Met Asp Thr Ala Ser Met Leu Asp Glu Ala Ile Arg Tyr Thr Lys
           35                  40                  45
   Phe Leu Lys
        50
<210> 8
   <211> 26
   <212> DNA
   <213> Artificial Sequence
<220>
   <223> Description of Artificial Sequence:primer for amplification of IND1 genomic region of cDNA
<400> 8
   gatgaaaatg gaaaatggta tgtata 26
<210> 9
   <211> 23
   <212> DNA
   <213> Artificial Sequence
<220>
   <223> Description of Artificial Sequence:primer for amplification of IND1 genomic region of cDNA
<400> 9
   gttcatcagg gttgggagtt gtg 23